# EUROPEAN PATENT APPLICATION

(11) **EP 2 727 578 A2**
(43) Date of publication of application: **07.05.2014**
(21) Application number: 14152657.4
(22) Date of filing: 30.04.2009
(51) Int. Cl.: A61K 8/34, A61K 8/36, A61K 8/40, A61K 8/49, A61K 8/73, A61K 8/81, A61Q 3/00, A61Q 5/00, A61Q 19/00, A61K 8/41, A61Q 5/04, A61Q 5/06, A61Q 5/10, A61Q 5/12, A61Q 13/00, C11D 3/40

(54) **Compositions and methods incorporating photocatalysts**

(30) Priority: 16.05.2008 US 53831 P
(62) Divisional of application: 09747162.7
(71) Applicant: The Procter & Gamble Company, Cincinnati, OH 45202 (US)
(72) Inventor: Schmidt Baker, Ellen, Cincinnati, OH Ohio 45202 (US); Constantinides, Ioannis, Cincinnati, OH Ohio 45202 (US); Dunbar, James Charles, Cincinnati, OH Ohio 45202 (US); Felts, Timothy James, Cincinnati, OH Ohio 45202 (US); Mueller, William Richard, Cincinnati, OH Ohio 45202 (US); Murphy, Bryan Patrick, Cincinnati, OH Ohio 45202 (US); Willey, Alan David, Cincinnati, OH Ohio 45202 (US)
(74) Representative: Holmes, Rosalind

(57) **Abstract**

A personal care composition including an active material having functional groups capable of covalent attachment to a substrate in the presence of an acid or a base, a photocatalyst capable of generating an acid or a base upon exposure to light, and a vehicle. A method for treating hair is also disclosed. The method includes the steps of applying at least one active material having functional groups to the substrate, applying a photocatalyst to the substrate, and exposing the photocatalyst and the at least one active material to light for forming covalent attachments between the functional groups and constituent groups on the substrate.

## Description

### FIELD OF INVENTION

A composition for and method of covalent modification of surface properties of a hair, comprising an active material having functional groups capable of covalent attachment to a substrate in the presence of an acid or a base, a photocatalyst capable of generating an acid or a base upon exposure to light, and a vehicle.

### BACKGROUND OF THE INVENTION

The overall properties of a material are controlled in significant part by the surface properties and the bulk properties of the material. The surface properties of a material are largely controlled by the surface chemistry and surface structure of the material. The bulk properties of a material are largely controlled by the bulk chemistry and bulk structure of the material. It is sometimes desirable to modify the surface chemistry and/or surface structure of a material in order to produce certain surface properties. In addition, it is sometimes desirable to modify the bulk chemistry and/or bulk structure of a material in order to produce certain bulk properties.

Surface modifications generally fall into two categories: (1) chemically or physically altering the atoms, compounds, or molecules in the existing surface (e.g., treatment, etching, chemical modification), or (2) over-coating the existing surface with a material having a different composition and/or structure (e.g., coating, grafting, thin film deposition). It may be desirable to modify the surface of a material by forming a covalent bond between functional groups on the material surface and complementary functional groups in an active material. It may be particularly desirable to covalently modify the surface of certain materials with active materials in order to modify the surface properties. It may also be desirable to modify the surface of a material by locally forming an active material on the material surface by reacting one or more active components to create covalent bonds between the one or more active components. An active material including active components covalently bound together may remain localized to the material surface by chemical or physical phenomena such as, for example, adsorption, absorption, electrostatic interaction, frictional interaction, steric interaction, and/or size exclusion effects. It may additionally be desirable to modify the bulk of a material by forming active material in a similar manner within the bulk of the material. Materials suitable for surface and/or bulk modification include, for example, physiological materials such as hair.

Hair is exposed to a variety of chemical and physical environments. For example, common hair care practices often include one or more of washing, blow drying, brushing, coloring, perming, relaxing, and styling. These activities may result in the loss of the natural luster and texture that characterizes healthy hair. Moreover, environmental factors may add to these effects and substantially contribute to weathered or damaged hair.

Hair is naturally protected from mechanical, chemical, and environmental mediated damage by the fiber cuticle surface membrane ("FCSM"). The FCSM comprises the outermost surface layer of hair fibers and includes protein and lipid components. The FCSM functions as a highly resistant, hydrophobic, surface-protective barrier to mechanical, chemical and environmental factors that would otherwise substantially contribute to hair damage. The FCSM comprises a surface lipid mono-layer, sometimes referred to as the F-layer, covalently bound to an underlying layer of heavily cross-linked keratinous protein, sometimes referred to as the epicuticle. The F-layer comprises predominately fatty acids such as 18-methyl-eicosanoic acid ("18-MEA") bound to the epicuticle through thioester linkages formed between the thiol groups on the cysteine residues in the keratin and other proteins in the epicuticle and the carboxyl group on the 18-MEA or other fatty acid. The F-layer gives hair fibers a hydrophobic surface, which in part facilitates the shiny luster, silky texture and smoothness of healthy hair.

Skin is naturally protected from mechanical, chemical, and environmental mediated damage by the stratum corneum. The stratum corneum is the outermost layer of epidermis. Despite differences in microstructure, the F-layer and the stratum corneum both possess similar protective functions for hair and skin respectively. However, mechanical, chemical and environmental factors may result in loss of at least a portion of the F-layer and the stratum corneum. For example, during permanent hair coloring, the combinations of hydrogen peroxide, ammonia and high pH may remove at least a portion of the protective F-layer, allowing for additional oxidation of the underlying hair surface, which may cause irreversible physiochemical changes in the hair fibers. Repeated colorings may cause the F-layer to completely disappear from the surface of hair fibers. As a result, the previously hydrophobic hair fiber surfaces may become hydrophilic because the keratinous epicuticle is exposed to the surface when the F-layer is lost. The natural protective and lubricating properties of the hair fiber surface are consequently diminished, and hair may feel dry, rough, frizzy, become difficult to brush and/or detangle, appear duller and less colorful, possess increased levels of static, and become substantially more susceptible to additional damage due to other mechanical, chemical and environmental factors.

Damage to the surface portions of these materials may lead to damage to the underlying bulk portions of the materials. This may ultimately result in substantial, and perhaps irreparable, damage to these materials. A variety of mechanical, chemical and environmental factors may contribute (solely or collectively) to hair and/or skin damage. For example, excessive exposure to sunlight, exposure to chlorine in pool water (and to a lesser extent in the water provided by municipal supply), exposure to other forms of water pollution, exposure to various forms of air pollution, frictional interactions between hair fibers, and frictional interaction between hair fibers or skin and other surfaces may contribute to hair damage.

A variety of approaches and products are available for treating hair in order to compensate for the loss of the F-layer, as well as underlying bulk damage. For example, leave-on and rinse-off hair conditioners attempt to compensate for F-layer loss by depositing various actives on the surfaces of hair fibers. Conditioners may include a variety of types of actives, for example, emollients, humectants, reconstructors (e.g., hydrolyzed proteins or peptides and free amino acids intended to penetrate the hair and strengthen its bulk structure through crosslinking), pH regulators, detanglers, thermal protectors (e.g., heat-absorbing polymers), glossers (e.g., silicones such as dimethicone or cyclomethicone), essential oils and fatty acids (e.g., as sebum substitutes), surfactants (e.g., moieties having hydrophobic and cationic functionality), and/or lubricants (e.g., panthenol). In addition, hair conditioners may include sequestrants/chelators, antistatic agents, rheology modifiers, emulsifiers, feel agents, fillers and/or preservatives. Hair conditioners function by depositing hydrophobic actives (e.g., petrolatum, dimethylsiloxanes, fatty alcohols, fatty acids, and/or hydrophobic quaternary ammonium salts) on the surface of hair fibers or skin. The actives are deposited in their active state and adhere to the surfaces through physical or physiochemical means such as, for example, absorption, adsorption, hydrogen bonding, ionic bonding, other electrostatic interaction, and/or other transient non-covalent association. Consequently, as a result of the transient non-covalent association with the hair fiber or skin surfaces, these compositions may have a substantially limited active life because they may be substantially removed from hair or skin surfaces due to interactions such as washing, rinsing, or other mechanical, chemical, or environmental interactions during normal daily activities.

Accordingly, there exists a need for compositions and methods to compensate for F-layer loss from hair fibers that provides a more durable conditioning and protective benefit. Covalent modification of the surface properties of damaged hair is one example of such an approach. There is also a need to protect, repair, and/or strengthen these materials. Modification of the surface of a material by locally forming an active material on the material surface by reacting one or more active components to create covalent bonds between the one or more active components and modification the bulk of a material by forming active material in a similar manner within the bulk of the material are promising approaches.

### BRIEF SUMMARY

The present invention relates to a personal care composition characterized in that it comprises:
(a) an active material having one or more functional groups capable of covalent attachment to a substrate in the presence of an acid or a base to one or more complementary functional groups;
(b) a photocatalyst capable of generating an acid or a base upon exposure to light; and
(c) a physiological acceptable vehicle for dispersing or dissolving the active material and the photocatalyst for application of the composition to a substrate; and
wherein the vehicle is a physiological acceptable vehicle and the substrate is selected from the group consisting ofhair;
wherein the active material is selected from the group consisting of glycerin, ethylene carbonate, poly(acrylic acid), ethyl oxazoline, poly(styrene), poly(vinyl pyrrolidone-co-acrylic acid), butane tetracarboxylic acid, citric acid, poly(styrene sulfonate-co-acrylic acid), an ethyl ester of PVM/MA copolymer, ethoxylated poly(dimethylsiloxane), cyclodextrin, cyclodextrin derivative, and combinations thereof;
wherein the photocatalyst is an aromatic hydroxyl compound.

The present invention also relates to a method for treating a substrate comprising:
applying at least one active material to the substrate, the active material having one or more functional groups, and the substrate having one or more complementary functional groups;
applying to the substrate at least one photocatalyst capable of generating an acid or base on exposure to light; and
exposing the photocatalyst and the at least one active material to light for forming covalent attachments between the one or more functional groups of the at least one active material and a reagent selected from the group consisting of a second active material, a substrate and a combination thereof;
wherein the active material is selected from the group consisting of glycerin, ethylene carbonate, poly(acrylic acid), ethyl oxazoline, poly(styrene), poly(vinyl pyrrolidone-co-acrylic acid), butane tetracarboxylic acid, citric acid, poly(styrene sulfonate-co-acrylic acid), an ethyl ester of PVM/MA copolymer, ethoxylated poly(dimethylsiloxane), cyclodextrin, cyclodextrin derivative, and combinations thereof;
wherein the photocatalyst is an aromatic hydroxyl compound.

The present invention also relates to a mechanism of use comprising:
(a) a reagent solution is provided that includes a reagent being an active component, and a photo acid catalyst;
(b) the reagent solution is applied to a substrate being hair
(c) the components of the reagent solution deposit on the surface of the substrate;
(d) the system comprising the reagent solution and the substrate is exposed to light and the light causes the deprotonation of the photocatalyst;
(e) a photoacid-catalyzed esterification reaction occurs between the reagent and the substrate surface;
(f) un-reacted catalyst, reagent, and protons diffuse from the substrate surface and are removed from the system;
(g) the modified/functionalized substrate is dried;
(h) the modified/functionalized substrate is washed and rinsed and the modified/functionalized substrate substantially retains the covalently bound reagent after washing and rinsing.

### BRIEF DESCRIPTION OF THE DRAWINGS

Various embodiments described herein may be understood by reference to the following description, taken with the accompanying drawings as follows.
**FIG. 1** is a schematic diagram that illustrates damage to the FCSM of a hair fiber comprising a keratinous epicuticle portion covalently bound to 18-MEA by way of thioester bonds.
**FIG. 2** is a schematic diagram that illustrates one non-limiting embodiment of the compositions and methods described herein for treating physiological substrates such as hair with an active component and a photocatalyst.
**FIG. 3** and **4** are schematic diagrams that illustrate one non-limiting embodiment of the compositions and methods described herein for treating physiological substrates such as hair with an active component and a photocatalyst.
**FIG. 5** is a schematic representation of one non-limiting embodiment of a mechanism of action of the compositions and methods described herein where a substrate surface is covalently modified.
**FIG. 6** is a schematic representation of one non-limiting embodiment of the compositions and methods described herein where a porous substrate material is treated with an active material capable of forming a secondary active material.
**FIG. 7** is a graph comparing the contact angle measurements of various examples presented herein and hair of various natures.

### DETAILED DESCRIPTION OF THE INVENTION

While the specification concludes with claims which particularly point out and distinctly claim the present invention; it is believed that the present invention will be better understood from the following description of various non-limiting embodiments.

The articles "a," "an," and "the" are used herein to refer to one or more than one (i.e., to at least one) of the grammatical objects of the article. By way of example, "a component" means one or more components. All percentages, parts and ratios are based upon the total weight of the compositions of the present invention, unless otherwise specified. All such weights as they pertain to listed ingredients are based on the active level and, therefore, do not include solvents or byproducts that may be included in commercially available materials, unless otherwise specified. As used herein, the term "functional group" means an atom or group of associated atoms that, at least in part, defines the structure and determines the properties of a particular family of chemical compounds. A functional group may be a region on or in a molecule or material that is a site of specific chemical reactivity compared to other regions of the molecule or material. Functional groups generally have characteristic properties and may control, in part, the reactivity of a molecule as a whole. Functional groups include, but are not limited to, hydroxyl groups, thiol groups, carbonyl groups, carboxyl groups, sulfonate groups, sulfide groups, ether groups, halogen atoms, amino groups, cyano groups, nitro groups, and the like. Compounds that are generally classified (structurally and/or functionally) according to functional groups include, but are not limited to, alkanes, alkenes, alkynes, aromatic compounds, halides, alcohols, ethers, esters, amines, imines, imides, carboxylic acids, amides, acid halides, acid anhydrides, nitriles, ketones, aldehydes, carbonates, peroxides, hydroperoxides, carbohydrates, acetals, epoxides, sulfonic acids, sulfonate esters, sulfides, sulfoxides, thioethers, thiocyanates, disulfides, phosphonic acids, phosphate esters, phosphines, azides, azo compounds, nitro compounds, nitrates, nitriles, nitrites, nitroso compounds, thiols, cyanates, and isocyanates, for example. The terms "active material", "active component", "active compound", and combinations and modifications of these terms, as used herein means substances to be applied to a substrate to modify the surface and/or bulk properties of the substrate material. These terms may be used interchangeably. Substrate surface properties may include, for example, surface hydrophobicity/hydrophilicity, oleophobicity/oleophilicity, color, optical properties, absorptivity, adsorptivity, bonding capability, brightness, dullness, frictional resistance, stain resistance, surface texture, odor, washability, wettability, elasticity, plasticity, and rigidity. Substrate bulk properties may include, for example, tensile strength, rigidity, absorptivity, elasticity, plasticity, and biological activity. Active materials may include compounds having one or more functional groups capable of covalent attachment in the presence of an acid or a base to one or more complementary functional groups present at the surface or in the bulk of a substrate. Active materials may also include compounds capable of forming covalent bonds between molecules in the presence of an acid or a base, for example, monomers capable of acid or base catalyzed polymerization. A "cosmetically active material" is an active material suitable for use in a personal care product without undue toxicity, incompatibility, instability, allergic response, and the like. The term "monomer" as used herein means a compound that may be covalently bonded to other monomers (that may have the same or different chemical structures) to form a polymer or copolymer. The term "polymer" (and "copolymer") as used herein means a compound comprising a plurality of monomers. Accordingly, as used herein the term polymer includes dimers, trimers, oligomers, and the like. As used herein, the terms "modify", "modification", "functionalize" or "functionalization", with regard to a substrate, refers to (1) covalently attaching an active component to the substrate surface, (2) covalently attaching an active component to the substrate in the bulk of the substrate material, (3) forming covalent bonds between two or more active components (which may be the same or different chemical moieties) where the resultant secondary active material localizes to the substrate surface, and/or (4) forming covalent bonds between two or more actives (which may be the same or different chemical moieties) where the actives are present within the bulk of the substrate. The term "suitable for application to human hair" and "suitable for application to human skin" as used herein means that the compositions or components thereof so described are suitable for use in contact with human hair and the scalp and skin without undue toxicity, incompatibility, instability, allergic response, and the like.

One object of the compositions and methods described herein is to provide for the modification of the surface and/or bulk properties of a substrate by covalently attaching an active material to the surface of the substrate. Another object of the compositions and methods described herein to provide for the modification of the surface and/or bulk properties of a substrate by treating the substrate with an active compound capable of reacting with itself to form covalent bonds between two or more molecules of the active compound thereby forming a secondary active material. It is still another object of the compositions and methods described herein to provide for the functionalization of the surface of a substrate by covalently attaching active material to the surface of the substrate. In order to achieve effective treatment, it is occasionally desirable to initially attach onto a substrate a material that contains multiple similar functional groups in its molecule, followed by another step of attaching another material/benefit agent by reacting with the initially attached material. This is especially useful if the substrate contains only limited density of functional groups that are able to react with a benefit agent towards a chemical bond. For example, initial attachment of malic acid (2-hydroxy-1,4-dibutanoic acid) onto a substrate increases the reactivity of the substrate by a factor of two towards subsequent attachment of an active. It is yet another object of the compositions and methods described herein to provide for such modification/functionalization in a manner that is readily amenable to health and safety regulations, and which may be readily implemented in a personal care product and/or a consumer care product space.

The various embodiments relate, in general, to compositions and methods for treating a substrate. As used herein, the term "substrate" means any material for which it is useful to treat the surface and/or bulk with the compositions and methods described herein. As claimed herein the substrate is hair. The terms "substrate" and "material" may be used interchangeably in the context of substances to be modified by the compositions and methods described herein.

In various embodiments, the compositions described herein include an active component that can modify a substrate in the presence of an acid or a base, a photocatalyst capable of generating an acid or a base upon exposure to light, and a suitable vehicle, which may optionally be a physiological acceptable vehicle. In various embodiments, the compositions described herein may also include one or more optional components, including surfactants, emulsifiers, oxidants, reductants, pH regulators, emollients, humectants, proteins, peptides, amino acids, additive polymers, glossers, essential oils and/or fatty acids, lubricants, sequestrants/chelators, antistatic agents, rheology modifiers, feel agents, fillers, preservatives, perfumes, other functional components, or combinations thereof.

In various embodiments, the methods described herein include treating a substrate by forming one or more covalent bonds between an active component and/or the substrate, where the covalent bond is formed in the presence of an acid or base generated by a photocatalyst upon exposure to light. In various embodiments, the methods described herein include treating a substrate by forming one or more covalent bonds between two or more active component molecules, where the covalent bond is formed in the presence of an acid or base generated by a photocatalyst upon exposure to light and the active material localizes to the surface and/or bulk of the substrate. As used herein, the term "molecule" means a sufficiently stable group of at least two atoms in a definite arrangement held together by chemical bonds. Accordingly, the term molecule includes, but is not limited to, neutral molecular compounds, polyatomic ions, radical species, biomolecules, monomers, dimers, trimers, oligomers, polymers, and the like.

In various embodiments, the methods described herein include treating a substrate by preparing and covalently bonding a compound to the substrate, or forming covalent bonds between compounds on the substrate surface or in the substrate bulk, in *situ,* by providing a substrate, providing one or more reagents, providing a photocatalyst, and exposing the photocatalyst to light in the presence of the substrate and the one or more reagents, where the photocatalyst generates an acid or a base, the acid or the base catalyzes reaction between the one or more reagents and/or reaction between the one or more reagents and the substrate, and where the reaction(s) forms covalent bonds. In various embodiments, the methods described herein include providing a system including a substrate, an active component that can modify a substrate in the presence of an acid or a base, and a photocatalyst capable of generating an acid or a base upon exposure to light, and exposing the system to light.

Generally, covalent attachment of active components on substrates such as hair and skin, for example, often proves difficult to achieve. This is especially true in the presence of water, which may rapidly degrade reactive moieties before substrate functionalization occurs. Moreover, aqueous media are known to chemically facilitate hydrolysis and oxidation reactions that may compete against covalent attachment of active components to substrates. This may pose particular problems, for example, in personal care products where water is often used as a physiologically acceptable vehicle. Consumer care products also often use water in a variety of capacities, most notably as a solvent.

In addition, substrates such as, for example, hair, may not contain particularly reactive chemical functional groups on the surface that would readily react with active components to form covalent bonds. This relatively low substrate surface reactivity may result in a reaction system that is outside the practical time frame of an apply-and-rinse environment (e.g., shampooing and conditioning hair). Furthermore, strict regulatory requirements concerning product safety and environmental protection increase the challenge of providing compositions and methods for treating a substrate such as, for example, hair, through covalent attachment of active components.

However, the various embodiments of the compositions and methods described herein are directed toward a photocatalyst technology that allows the use of light to promote a reaction such as, for example, the covalent attachment of an active component to a substrate or formation of covalent bonds between two or more active components *in situ* on the surface or in the bulk of a substrate material. The various embodiments may be used, for example, to promote the covalent attachment of long-chain alkyl groups to damaged hydrophilic hair in order to replenish and/or fortify the normally hydrophobic character of these substrates. Furthermore, the various embodiments may be used, for example, to locally polymerize monomers on the surface and/or in the bulk of substrate materials in order to modify the surface and/or bulk properties of a material.

In various embodiments, covalent attachment may yield a variety of substantial benefits to individuals that possess damaged hair. For example, hair conditioning benefits may include, among others, improved feel, lower friction, easier combing/brushing, reduced dryness, increased smoothness, decreased frizziness, increased shine, decreased levels of static, and improved protection against damage due to other mechanical, chemical and environmental factors. Skin conditioning benefits may include, among others, decreased dryness, decreased redness, decreased itchiness, decreased flaking, and improved texture and smoothness. At least some of these benefits may be imparted by increased or targeted deposition of actives resulting from the surface modification via covalent attachment. The benefits imparted by the compositions and methods described herein are potentially more durable because a non-labile covalent bond is employed, which is generally stronger and more stable relative to the absorption, adsorption, hydrogen bonding, ionic bonding, other electrostatic interactions, and/or other transient non-covalent associations employed in prior conditioners to deposit or apply active components onto hair and/or skin. This may substantially reduce the frequency of application and reapplication encountered with prior conditioners.

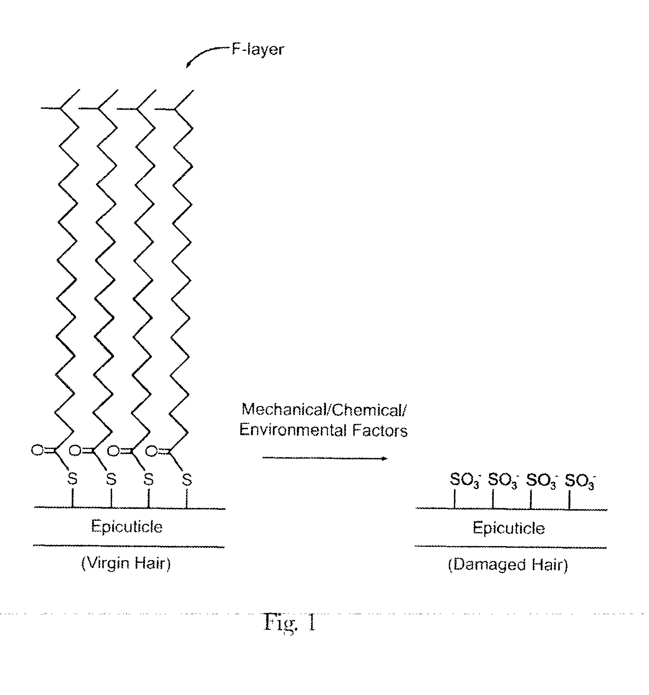

Various embodiments of the compositions and methods described herein provide for the covalent attachment of active components to substrates, which may be described as an approach toward repairing and/or fortifying the hair F-layer or skin stratum corneum for example. In the context of hair, and not to be bound or otherwise limited by theory, the F-layer of virgin hair may be stripped from the hair fiber by processes mediated by various mechanical, chemical, and/or environmental factors as illustrated in **FIG.1****.** These processes may include, for example, the oxidative and hydrolytic reactions commonly encountered during permanent hair coloring and permanent waving processes.

**FIG.1** is a schematic diagram that illustrates the FCSM of a hair fiber comprising a keratinous epicuticle portion covalently bound to 18-MEA by way of thioester bonds between the carboxyl group on the 18-MEA and the thiol group on cysteine residues in the keratin protein in the epicuticle. Hydrolytic and/or oxidative processes (for example, due to the combinations of hydrogen peroxide, ammonia and high pH commonly encountered during permanent hair coloring and permanent waving processes), as well as other mechanical, chemical, and environmental factors, may remove at least a portion of the F-layer by cleaving the cysteine-lipid thioester bond, leaving exposed epicuticle comprising sulfonate groups on the cysteine residues.

The anionic sulfonate groups on the cysteine residues at the surface of the epicuticle render the surface of any damaged hair fibers hydrophilic, which may result in the undesirable properties of damaged hair. Moreover, it has been observed that the more hydrophilic (and consequently the more damaged) the hair fibers, the lower the deposition of prior hydrophobic conditioning actives (such as, for example, dimethylsiloxanes, fatty alcohols and acids, and quaternary amines) by non-covalent interactions and associations. Accordingly, the compositions and methods described herein provide an attractive approach for treating such damaged substrates.

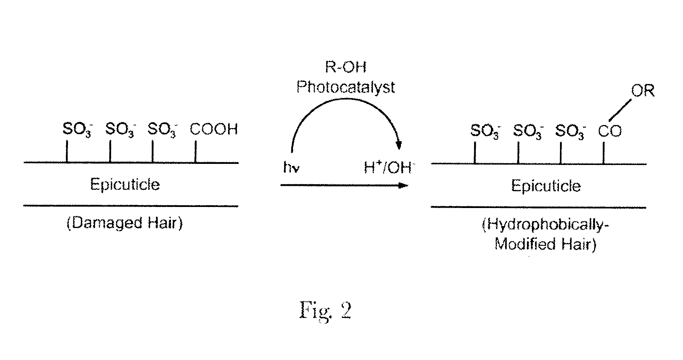

**FIG.2** schematically illustrates one non-limiting embodiment of the compositions and methods described herein for treating substrates. A composition comprising an active component having a hydroxyl group (R-OH) and a photocatalyst capable of generating an acid or a base upon exposure to light is provided in the presence of a substrate comprising surface sulfonate and carboxyl groups. The photocatalyst is exposed to light, which causes the photocatalyst to form an acid or a base. The acid or base catalyzes the formation of a covalent ester bond between the hydroxyl group on the active material and the carboxyl group on the substrate.

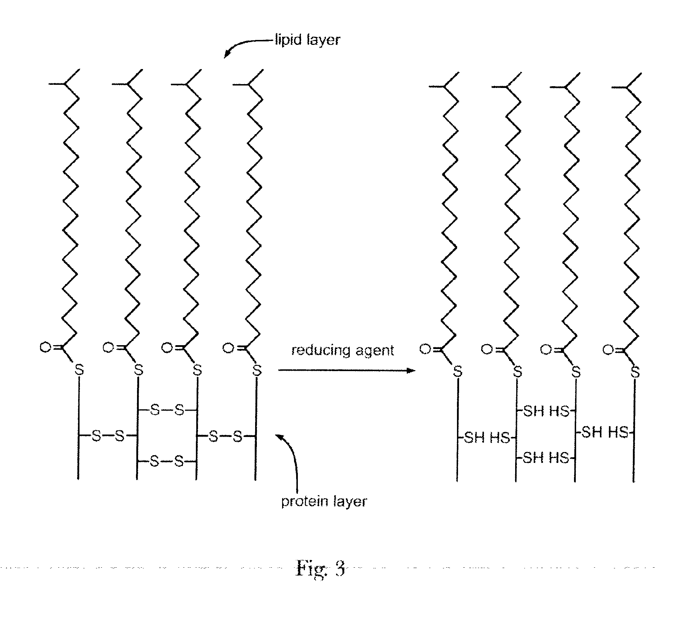

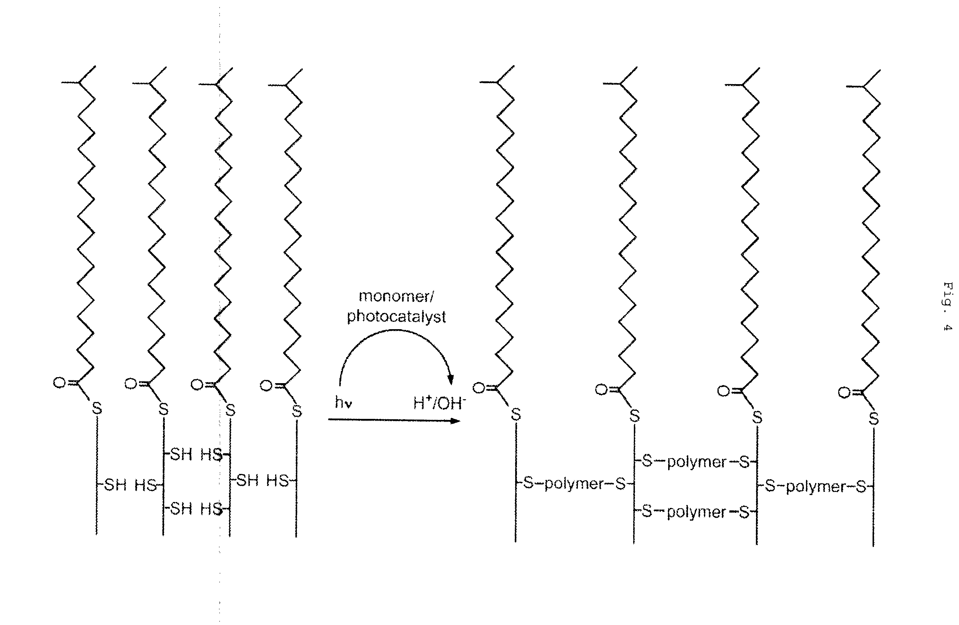

**FIG.3** and **FIG.4****,** viewed together, schematically illustrate one non-limiting embodiment of the compositions and methods described herein for treating substrates. A portion of a hair fiber comprising a lipid layer (F-layer) and a protein layer (epicuticle) is shown. The protein layer comprises structural proteins such as, for example, keratin having disulfide bonds between cysteine residues. The hair may be treated with a reducing agent to break the disulfide bonds and form respective thiol groups. The hair may be further treated with an active component comprising one or more compounds capable of reaction to form covalent bonds between the one or more active component compounds and/or between the one or more active component compounds and the thiol groups. The hair fiber is also treated with a photocatalyst. The one or more active components and the photocatalyst penetrate the surface of the hair fiber substrate. The hair fiber substrate treated with the one or more active components and photocatalyst is exposed to light of suitable wavelength to activate the photocatalyst and catalyze reaction between the one or more active components within the hair fiber substrate and the thiol groups.

In various embodiments, the active components may be one or more monomers capable of polymerizing in the presence of acid or base. The hair fibers are treated with a composition comprising photocatalyst and monomer, which at least partially penetrates the fiber. Upon exposure to light, the photocatalyst is activated thereby generating acid or base, which catalyzes the polymerization of the monomer, thereby forming a polymer *in situ,* which may optionally attach to the hair fiber by way of covalent bonds formed between the thiol groups and the polymer.

In other embodiments (not shown), the polymer does not covalently attach to the hair fiber. For example, the polymer formed *in situ* may be physically immobilized on the surface of the hair fiber or within pores in the hair fiber. The polymer formed *in situ* may also be associated with the hair fiber by a physical and/or chemical interaction such as, for example, adsorption, absorption, electrostatic interaction, frictional interaction, steric interaction, and/or size exclusion effects with the surface and/or bulk of the substrate.

In various embodiments, the monomer may be styrene or a styrene derivative such as, for example, α-methyl styrene. The monomer may also comprise mixtures of different monomers such that the *in situ* polymerization (on the surface and/or in the bulk of the substrate) produces copolymer.

In various embodiments of the compositions and methods described herein, the photocatalyst may be a photoacid that deprotonates upon exposure to light. The proton (which may be solvated, e.g., in the form of a hydronium ion) may catalyze the formation of a covalent bond through an esterification reaction or a thioesterification reaction, for example. In various embodiments of the compositions and methods described herein, the photocatalyst may be a photobase that generates hydroxide anion upon exposure to light. The hydroxide anion may catalyze the formation of a covalent bond through an esterification reaction or thioesterification reaction, for example. In various embodiments, the mechanism of action of a photoacid or photobase is not limited to an Arrhenius-type or Brønsted-Lowry type acid or base system, but rather may also include a Lewis-type acid or base that is catalytically activated upon exposure to light. The compositions and methods described herein are not limited in this context.

Esterification reactions are generally reversible. In relatively neutral media, such as water, the reversible esterification reaction may not thermodynamically favor the formation of the ester bond and water, as opposed to the reverse reaction of hydrolysis of the ester bond to respective hydroxyl and carboxyl containing moieties. Thioesterification systems generally behave in an analogous manner. Thus, the formation of covalent bonds between active components and substrates in prior systems, for example in prior conditioners, was impracticable in the context of treating substrates such as, for example, hair.

In addition, acid or base catalysis of esterification or thioesterification reactions are generally impracticable in the context of personal care products because it is difficult to generate sufficient acid or base concentration at the surface or within the bulk of the substrate without having relatively high or relatively low pH. The use of products having relatively high or relatively low pH is generally inappropriate because such acidic and caustic substances may be physiologically unacceptable.

The compositions and methods described herein overcome these limitations. The use of a photocatalyst allows for the co-localization of the catalyst and an active component at a substrate surface or within the bulk of the substrate material. The photocatalyst however is not activated until it is exposed to light. Photoacid catalysts, for example, exhibit a decrease in pKa upon exposure to light of suitable wavelength. Photobase catalysts, for example, may exhibit an increase in pKb upon exposure to light of a suitable wavelength. The respective increase in acid or base strength upon exposure to light results in a localized increase in proton or hydroxide concentration at the substrate surface which facilitates rapid esterification or thioesterification, for example. Moreover, because the proton or hydroxide concentration is localized at the substrate surface for a short period of time (before diffusing into the surrounding medium), bulk pH may be essentially unaffected by the photocatalytic reaction and may remain close to neutral, given the quantity of the photocatalyst used. This is advantageous for physiological applications such as, for example, in personal care products and in various consumer care product applications. In addition, the transient localized nature of the acidic or basic catalysis also contributes to the stability of the covalent bond formed during the process in cases where the covalent bond is sensitive to high or low pH. Therefore, photocatalysis of the reactions forming ester and/or thioester covalent bonds between active components and substrates in the various embodiments of the compositions and methods described herein provides for an efficient, controllable, stable and physiologically acceptable approach to substrate treatment such as, for example, F-layer repair and/or fortification in hair.

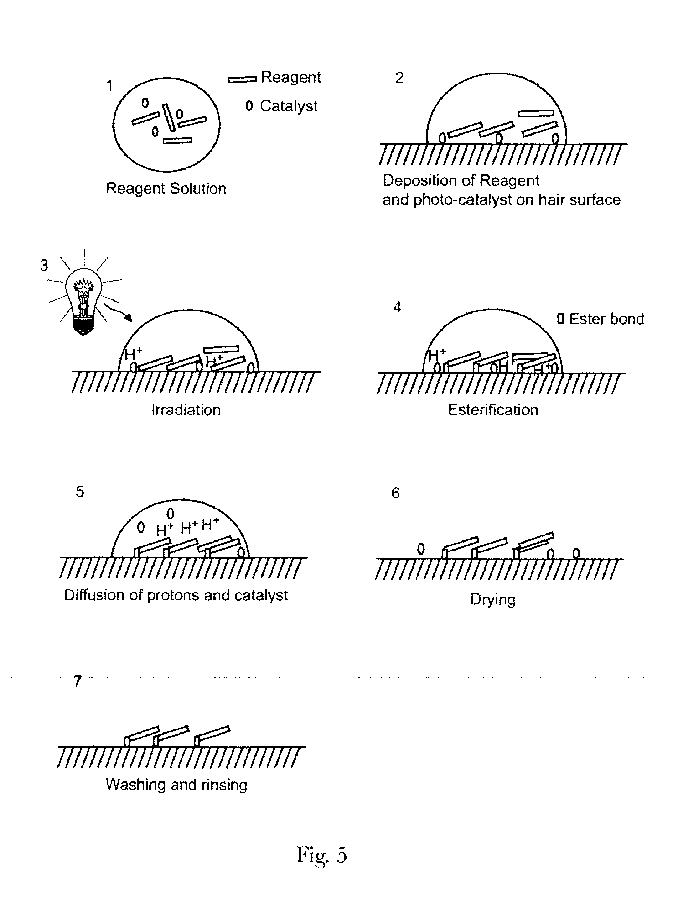

**FIG. 5** is a schematic representation of one non-limiting embodiment of a mechanism of use of the compositions and methods described herein in the context of a photoacid catalyst. In the first step, a reagent solution is provided that includes a reagent, which may be an active component, and a photoacid catalyst. The reagent solution may comprise a shampoo, a conditioner, other personal care product or a consumer care product. In the second step, the reagent solution is applied to a substrate, which may be hair, for example. The components of the reagent solution deposit on the surface of the substrate. In the third step, the system comprising the reagent solution and the substrate is exposed to light. The light causes the deprotonation of the photocatalyst. In the fourth step, a photoacid-catalyzed esterification reaction occurs between the reagent and the substrate surface. In the fifth step, un-reacted catalyst, reagent, and protons diffuse from the substrate surface and are removed from the system. In the sixth step, the modified/functionalized substrate is dried. In the seventh step, the modified/functionalized substrate is washed and rinsed. The modified/functionalized substrate substantially retains the covalently bound reagent after washing and rinsing.

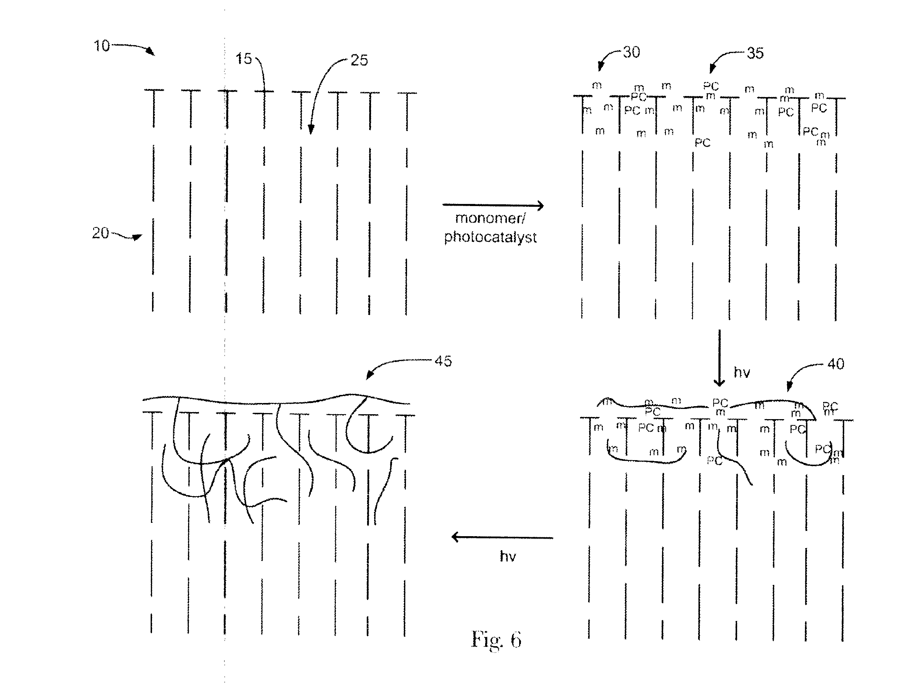

**FIG. 6** is a schematic representation of one non-limiting embodiment of the compositions and methods described herein. A porous substrate material 10 is provided. The substrate material 10 includes a substrate surface 15 and a bulk portion 20 having pores 25. The substrate material 10 is treated with a composition comprising an active material 30 and a photocatalyst 35. The active material 30 may comprise molecules capable of reacting together in the presence of an acid or a base to form a secondary compound. For example, the active material 30 may comprise one or more types of monomer capable of reacting to form polymer or copolymer in the presence of acid or base. The active material 30 and the photocatalyst 35 penetrate, at least in part, the surface 15 of the substrate 10 into the bulk portion 20 through pores 25. The substrate 10 is exposed to light of suitable wavelength to activate the photocatalyst 35, which generates acid or base to catalyze the reaction of the active material 30 on the surface 15 and/or in the bulk portion 20. As a result, secondary active material 40 forms on the surface 15 and/or in the bulk portion 20 of substrate material 10. Secondary active material 40 may comprise dimers, trimers, oligomers, polymers, copolymers or combinations thereof, for example. The secondary active material 40 may form a polymer network 45 that may modify the surface and/or bulk properties of the substrate material 10.

The secondary active material formed according to the photocatalyzed acid or base mechanism described herein may localize to the surface and/or bulk of the substrate material. In various embodiments, the localization may be a result of covalent attachment of the secondary active material to the substrate material. In other embodiments, the localization may be a result of non-covalent chemical or physical interactions between the secondary active material and the surface and/or bulk of the substrate material. For example, **FIG. 6** illustrates a secondary active material comprising a polymer network that is immobilized on the surface and partially in the bulk of a substrate material due to the physical formation of the polymer within pores located in the material. In other embodiments (not shown in **FIG. 6****),** the secondary active material formed according to the photocatalyzed acid or base mechanism described herein may localize on the surface of a substrate and/or in the bulk of the substrate due to interactions such as adsorption, absorption, electrostatic interaction, frictional interaction steric interaction, and/or size exclusion effects. This allows for the manipulation of various material properties such as, for example, porosity of the treated substrate.

In various embodiments the secondary active material formed according to the photocatalyzed acid or base mechanism described herein may localize on the surface of a substrate and/or in the bulk of the substrate due to changes in the properties of the active material after the formation of covalent bonds between two or more active material molecules. For example, where the active material comprises a monomer/polymer system, the active material may be polymerized and/or crosslinked on the surface of a substrate. The polymerization and/or crosslinking may change the solubility of the active material in the reaction medium, which may facilitate the deposition of the secondary active material onto the substrate surface. In this manner, a surface layer of secondary active material may form on the substrate surface thereby modifying the surface properties. This may result in the encapsulation of constituent fibers in fibrous substrates such as, for example, hair and fabrics. In various embodiments (not shown in **FIG. 6****)** the active material formed according to the photocatalyzed acid or base mechanism described herein may also be covalently bonded to the substrate (surface and/or bulk) through a photocatalyzed acid or base mechanism as described herein.

The compositions and methods described herein facilitate *in situ* and localized modification of material properties in a controlled manner. The active components are covalently altered (e.g., by the formation of covalent bonds between active components to form a secondary active material and/or between active components and a substrate material) in a photoacid or photobase reaction system.

The substrate to be modified may be treated by spraying, soaking, spreading, coating, rinsing, or any other suitable means of introducing the composition onto the surface of the substrate or into the bulk of the substrate material. In various embodiments, it is important to ensure the entire surface of the substrate is wetted by reagent solution in order to ensure sufficient modification of the substrate surface and/or bulk. If the active material is at least partially insoluble in the vehicle, it is important to maximize contact between the active and the substrate by, for example, minimizing the drop size or particle size of the active in the vehicle. In various embodiments, it may be desired to introduce reagent solution onto only a single portion or multiple portions of a substrate surface. In other embodiments, it may be desired to irradiate only a single portion or multiple portions of a substrate surface with light of a wavelength suitable to activate the photocatalyst. The covalent modification only occurs on those areas of the substrate surface (and underlying bulk) that are both in contact with a reagent solution and irradiated with light of a wavelength suitable to activate the photocatalyst. This allows for control of the location and extent of the surface and/or bulk modification.

The acid or base photocatalytic covalent modification/functionalization mechanisms described herein may also be reversible. For example, substrate surfaces covalently modified or functionalized through esterification and/or thioesterification reactions may be contacted with an acidic aqueous surfactant solution. Alternatively, an alkaline surfactant solution may be employed. These solutions may facilitate the hydrolytic cleavage of the ester and/or thioester bonds attaching the active components to the substrate, thereby removing the active components.

This removability is limited to active component-substrate bonds that are reversible under the appropriate conditions. For example, in the case of photoacid-catalyzed esterification, the ester bond is formed when the reagent and the catalyst are present in the vicinity of the substrate and exposed to the appropriate light. The high concentration of protons at the moment of irradiation results in ester bond formation that remains intact because the generated protons diffuse rapidly into the bulk of the medium. The low content of the photoacid allows for subsequent stable and near-neutral pH of the bulk aqueous solution. Under these conditions the ester bond is hydrolyzed at a very slow rate. However, treatment with significantly lower (or significantly higher) pH aqueous solutions will more readily break the ester bonds resulting in the original unmodified substrate surface.

The removal of the covalently-attached active can also be achieved by treatment of the modified or functionalized substrate with a composition including a photocatalyst (photoacid or photobase). This allows for improved control over the timing of the removal of the active component from the substrate. This can be achieved if the photocatalyst is chosen so that it is unaffected by ambient light but can generate acid or base species under light of a specific wavelength provided by an appropriate device.

Each of the various components of the compositions and associated methods described herein, as well as preferred and optional components, are described in detail.

### Active Component

The active component or active material may be any chemical moiety that is capable of modifying/functionalizing a substrate in the presence of acid or base. Non-limiting examples of active materials include, but are not limited to, hydroxyl- or carboxyl-containing long-chain alkyl (straight- or branched-chain) moieties. In various embodiments, the active material may be one or more of a fatty acid, a fatty alcohol, a fatty amine, an aminosilicone, a polyvinyl alcohol, a polyvinyl alcohol-polyvinyl pyrrolidone copolymer, a polycaprolactone, an optical brightener, a humectant, a silanol, a dimethylsilicone functionalized with one or more of primary, secondary, carboxyl or hydroxyl functional groups.

In various embodiments, the active material preferably includes a substantial hydrophobic portion. However, in other embodiments, the active material may be hydrophilic. In various embodiments, the active material is one or more of a dye or other coloring agent or a perfume. In one embodiment the active component may be a perfume, that contains in its chemical structure a primary or secondary hydroxyl group, and/or an amine group, or a carboxyl group.

In various embodiments, the active material includes at least two active compounds capable of covalent attachment to a substrate in the presence of an acid or a base upon reaction between the at least two active compounds. In various embodiments, the active material forms secondary compounds, such as, for example, dimers, oligomers, polymers and combinations thereof, and, optionally, the secondary material has functional groups capable of covalent attachment to the substrate.

Non-limiting examples of additional active materials may include one or more of glycerin, stearyl alcohol, lauric acid, direct dye 243, ethylene carbonate, poly(acrylic acid), ethyl oxazoline, poly(vinyl pyrrolidone-co-acrylic acid), butane tetracarboxylic acid, citric acid, and poly(styrene sulfonate-co-acrylic acid).

The active material may be present in the compositions and methods described herein in an amount from 0.01 percent to 80 percent by weight relative to the total weight of the composition.

### Photocatalyst

The photocatalyst may be any acid, base (or conjugate thereof) having a pKa (or pKb) value that decreases (or increases) upon exposure to light. The light may be light of any suitable wavelength to result in the respective decrease or increase in pKa or pKb. For example the light may be ambient light, sunlight, incandescent light, fluorescent light, LED light, laser light, and the like. The light may fall within any classification along the electromagnetic spectrum, such as, for example, visible light, near or far ultraviolet light, or near or far infrared light.

In addition, the suitable light may be provided from any source capable of illuminating the substrate surface. For example, ambient sunlight, incandescent light, fluorescent light, and the like may provide light of suitable wavelength. Accordingly, the light may be provided by conventional sources such as lamps and portable or battery-powered lights. In addition, specific devices may be developed or adapted for use with the compositions and method described herein. For example, a hair brush configured to incorporate LEDs that provide light of a suitable wavelength may be used to covalently modify the surface of hair fibers. In various embodiments, a laser may be used to provide precise targeting of the covalent modification of substrate surfaces, for example.

In various embodiments, the photocatalyst is a photoacid such as, for example, an aromatic hydroxy compound, a sulfonated pyrene compound, an onium salt, a diazomethane derivative, a bissulfone derivative, a disulfuno derivative, a nitrobenzyl sulfonate derivate, a sulfonic acid ester derivative, a sulfonic acid ester of an N-hydroxyimide, or combinations thereof.

Photoacid catalysts may include, for example, hydroxy-substituted aromatics such as, for example, 8-hydroxyquinoline, 8-hydroxyquinoline sulfate, 8-quinolinol-1-oxide, 5-hydroxyquinoline, 6-hydroxyquinoline, 7-hydroxyquinoline, 5-iodo-7-sulfo-8-hydroxyquinoline, 5-fluoro-8-hydroxyquinoline, 5-fluoro-7-chloro-8-hydroxyquinoline, 5-fluoro-7-bromo-8-hydroxyquinoline, 5-fluoro-7-iodo-8-hydroxyquinoline, 7-fluoro-8-hydroxyquinoline, 5-chloro-8-hydroxyquinoline, 5,7-dichloro-8-hydroxyquinoline, 5-chloro-7-brono-8-hydroxyquinoline, 5-chloro-7-iodo-8-hydroxyquinoline, 7-chloro-8-hydroxyquinoline, 5-bromo-8-hydroxyquinoline, 5-bromo-7-chloro-8-hydroxyquinoline, 5,7-dibromo-8-hydroxyquinoline, 5-bromo-7-iodo-8-hydroxyquinoline, 7-bromo-8-hydroxyquinoline, 5-iodo-8-hydroxyquinoline, 5-iodo-7-chloro-8-hydroxyquinoline, 5,7-diiodo-8-hydroxyquinoline, 7-iodo-8-hydroxyquinoline, 5-sulfonic acid-8-hydroxyquinoline, 7-sulfonic acid-8-hydroxyquinoline, 5-sulfonic acid-7-iodo-8-hydroxyquinoline, 5-thiocyano-8-hydroxyquinoline, 5-chloro-8-hydroxyquinoline, 5-bromo-8-hydroxyquinoline, 5,7-dibromo-8-hydroxyquinoline, 5-iodo-8-hydroxyquinoline, 5,7-diiodo-8-hydroxyquinoline, 7-azaindole, 7-cyano-2-naphthol, 8-cyano-2-naphthol, 5-cyano-2-naphthol, 1-hydroxy-3,6,8-pyrenetrisulfonic acid, Trans-3-hydroxystilbene, 2-hydroxymethylphenol, or Pelargonidin.

Photoacid catalysts may include onium salts such as, for example, bis(4-tert-butylphenyl)iodonium perfluoro-1-butanesulfonate, diphenyliodonium perfluoro-1-butanesulfonate, diphenyliodonium-9,10-dimethoxyanthracene-2-sulfonate, diphenyliodonium hexafluorophosphate, diphenyliodonium nitrate, diphenyliodonium p-toluenesulfonate, diphenyliodonium triflate, (4-methylphenyl)diphenylsulfonium triflate, (4-methylthiophenyl)methyl phenyl sulfonium triflate, 2-naphthyl diphenylsulfonium triflate, (4-phenoxyphenyl)diphenylsulfonium triflate, (4-phenylthiophenyl)diphenylsulfonium triflate, thiobis(triphenyl sulfonium hexafluorophosphate), triarylsulfonium hexafluoroantimonate, triarylsulfonium hexafluorophosphate salt, triphenylsulfonium perfluoro-1-butanesulfonate, triphenylsulfonium triflate, tris(4-tert-butylphenyl)sulfonium perffuoro-1-butanesulfonate, tris(4-tert-butylphenyl)sulfonium triflate, bis(4-tert-butylphenyl)iodonium p-toluenesulfonate, bis(4-tert-butylphenyl)iodonium triflate, (4-bromophenyl)diphenylsulfonium triflate, (tert-butoxycarbonylmethoxynaphthyl)diphenylsulfonium triflate, (tert-butoxycarbonylmethoxyphenyl)diphenylsulfonium triflate, (4-tert-butylphenyl)diphenylsulfonium triflate, (4-chlorophenyl)diphenylsulfonium triflate, (4-fluorophenyl)diphenylsulfonium triflate, [4-[2-hydroxytetradecyl)oxy]phenyl]phenyliodonium hexafluoroantimonate, (4-iodophenyl)diphenylsulfonium triflate, (4-methoxyphenyl)diphenylsulfonium triflate, diphenyliodo hexafluorophosphate, diphenyliodo hexafluoroarsenate, diphenyliodo hexafluoroantimonate, diphenyl p-methoxyphenyl triflate, diphenyl p-toluenyl triflate, diphenyl p-isobutylphenyl triflate, diphenyl p-t-butylphenyl triflate, triphenylsulfonium hexafluorophosphate, triphenylsulfonium hexafluoroarsenate, triphenylsulfonium hexafluoroantimonate, triphenylsulfonium triflate, dibutylnaphthyl sulfonium triflate, diphenyliodonium trifluoromethanesulfonate, (p-tert-butoxyphenyl)phenyliodonium trifluoromethanesulfonate, diphenyliodonium p-toluenesulfonate, (p-tert-butoxyphenyl)phenyliodonium p-toluenesulfonate, triphenylsulfonium trifluoromethanesulfonate, (p-tert-butoxyphenyl)diphenylsulfonium trifluoromethanesulfonate, bis(p-tert-butoxyphenyl)phenylsulfonium trifluoromethanesulfonate, tris(p-tert-butoxyphenyl)-sulfonium trifluoromethanesulfonate, triphenylsulfonium p-toluenesulfonate, (p-tert-butoxyphenyl)diphenylsulfonium p-toluenesulfonate, bis(p-tert-butoxyphenyl)phenylsulfonium p-toluenesulfonate, tris(p-tert-butoxyphenyl)sulfonium p-toluenesulfonate, triphenylsulfonium nonafluorobutanesulfonate, triphenylsulfonium butanesulfonate, trimethyl-sulfonium trifluoromethanesulfonate, trimethylsulfonium p-toluenesulfonate, cyclohexylmethyl(2-oxocyclohexyl)-sulfonium trifluoromethanesulfonate, cyclohexylmethyl(2oxocyclohexyl) sulfonium p-toluenesulfonate, dimethylphenyl-sulfonium trifluoromethanesulfonate, dimethylphenyl-sulfonium p-toluenesulfonate, dicyclohexylphenylsulfonium trifluoromethanesulfonate, dicyclohexylphenylsulfonium p-toluenesulfonate, trinaphthylsulfonium trifluoromethane-sulfonate, cyclohexylmethyl(2-oxocyclohexyl)sulfonium trifluoromethanesulfonate, (2-norbornyl)methyl(2-oxocyclo-hexyl)sulfonium trifluoromethanesulfonate, ethylenebis-[methyl(2-oxocyclopentyl)sulfonium trifluoromethane-sulfonate], or 1,2'-naphthylcarbonylmethyltetrahydrothiophenium triflate.

Photoacid catalysts may include diazomethane derivatives such as, for example, bis(benzenesulfonyl)-diazomethane, bis(p-toluenesulfonyl)diazomethane, bis(xylenesulfonyl)diazomethane, bis(cyclohexylsulfonyl)-diazomethane, bis(cyclopentylsulfonyl) diazomethane, bis(n-butylsulfonyl)diazomethane, bis(isobutylsulfonyl)-diazomethane, bis(sec-butylsulfonyl)diazomethane, bis(n-propylsulfonyl) diazomethane, bis(isopropylsulfonyl)-diazomethane, bis(tert-butylsulfonyl) diazomethane, bis(n-amylsulfonyl)diazomethane, bis(isoamylsulfonyl)-diazomethane, bis(sec-amylsulfonyl)diazomethane, bis(tert-amylsulfonyl) diazomethane, 1-cyclohexylsulfonyl-1-(tert-butylsulfonyl)diazomethane, 1-cyclohexylsulfonyl-1-(tert-amylsulfonyl)diazomethane, or 1-tert-amylsulfonyl-1-(tert-butylsulfonyl)diazomethane.

Photoacid catalysts may include glyoxime derivatives such as, for example, bis-o-(p-toluenesulfonyl)-α-dimethylglyoxime, bis-o-(p-toluenesulfonyl)-α-diphenylglyoxime, bis-o-(p-toluenesulfonyl)-α-dicyclohexyl-glyoxime, bis-o-(p-toluenesulfonyl)-2,3-pentanedione-glyoxime, bis-o-(p-toluenesulfonyl)-2-methyl-3,4-pentane-dioneglyoxime, bis-o-(n-butanesulfonyl)-α-dimethylglyoxime, bis-o-(n-butanesulfonyl)-α-diphenylglyoxime, bis-o-(n-butanesulfonyl)-α-dicyclohexylglyoxime, bis-o-(n-butane-sulfonyl)-2,3-pentanedioneglyoxime, bis-o-(n-butanesulfonyl)-2-methyl-3,4-pentanedioneglyoxime, bis-o-(methanesulfonyl)-α-dimethylglyoxime, bis-o-(trifluoro-methanesulfonyl)-α-dimethylglyoxime, bis-o-(1,1,1-trifluoroethanesulfonyl)-α-dimethylglyoxime, bis-o-(tert-butanesulfonyl)-α-dimethylglyoxime, bis-o-(perfluorooctanesulfonyl)-α-dimethylglyoxime, bis-o-(cyclohexane-sulfonyl)-α-dimethylglyoxime, bis-o-(benzenesulfonyl)-α-dimethylglyoxime, bis-o-(p-fluorobenzenesulfonyl)-α-dimethylglyoxime, bis-o-(p-tert-butylbenzenesulfonyl)-α-dimethylglyoxime, bis-o-(xylenesulfonyl)-α-dimethyl-glyoxime, or bis-o-(camphorsulfonyl)-α-dimethylglyoxime.

Photoacid catalysts may include bissulfone derivatives such as, for example, bisnaphthylsulfonylmethane, bistrifluoromethylsulfonylmethane, Bismethylsulfonylmethane, bisethylsulfonylmethane, bispropylsulfonylmethane, bisisopropylsulfonylmethane, bis-p-toluenesulfonylmethane, bisbenzenesulfonylmethane, 2-cyclohexyl-carbonyl-2-(p-toluenesulfonyl)propane (β-ketosulfone derivative), 2-isopropyl-carbonyl-2-(p-toluenesulfonyl) propane (β-ketosulfone derivative).

Photoacid catalysts may include disulfono derivatives such as, for example, diphenyl disulfone or dicyclohexyl disulfone.

Photoacid catalysts may include nitrobenzyl sulfonate derivatives such as, for example, 2,6-dinitrobenzyl p-toluenesulfonate or 2,4-dinitrobenzyl p-toluenesulfonate.

Photoacid catalysts may include sulfonic acid ester derivatives such as, for example, 1,2,3-tris(methanesulfonyloxy) benzene, 1,2,3-tris(trifluoro-methanesulfonyloxy)benzene, or 1,2,3-tris(p-toluenesulfonyloxy)benzene.

Photoacid catalysts may include sulfonic acid esters of N-hydroxyimides such as, for example, N-hydroxysuccinimide methanesulfonate, N-hydroxysuccinimide trifluoromethanesulfonate, N-hydroxysuccinimide ethanesulfonate, N-hydroxysuccinimide 1-propanesulfonate, N-hydroxysuccinimide 2-propanesulfonate, N-hydroxysuccinimide 1-pentanesulfonate, N-hydroxysuccinimide 1-octanesulfonate, N-hydroxysuccinimide p-toluenesulfonate, N-hydroxysuccinimide p-methoxybenzenesulfonate, N-hydroxysuccinimide 2-chloroethanesulfonate, N-hydroxysuccinimide benzenesulfonate, N-hydroxysuccinimide 2,4,6-trimethyl-benzenesulfonate, N-hydroxysuccinimide 1-naphthalenesulfonate, N-hydroxysuccinimide 2-naphthalenesulfonate, N-hydroxy-2-phenylsuccinimide methanesulfonate, N-hydroxymaleimide methanesulfonate, N-hydroxymaleimide ethane-sulfonate, N-hydroxy-2-phenylmaleimide methanesulfonate, N-hydroxyglutarimide methanesulfonate, N-hydroxyglutarimide benzenesulfonate, N-hydroxyphthalimide methanesulfonate, N-hydroxyphthalimide benzenesulfonate, N-hydroxyphthalimide trifluoromethanesulfonate, N-hydroxyphthalimide p-toluenesulfonate, N-hydroxynaphthalimide methanesulfonate, N-hydroxynaphthalimide benzenesulfonate, N-hydroxy-5-norbornene-2,3-dicarboxyimide methanesulfonate, N-hydroxy-5-norbornene-2,3-dicarboxyimide trifluoromethanesulfonate, N-hydroxy-5-norbornene-2,3-dicarboxyimide p-toluenesulfonate, N-hydroxynaphthalimide triflate, N-hydroxy-5-norbornene-2,3-dicarboximide perfluoro-1-butanesulfonate.

In certain embodiments, the photocatalyst is 8-hydroxyquinoline, which may act as a photoacid catalyst in lower pH solutions or as a photobase catalyst in higher pH solutions. In certain other embodiments, the photocatalyst is 8-hydroxy-1,3,6-pyrentrisulfonic acid trisodium salt (D&C Green 8). In various embodiments, the photocatalyst is a photobase. Photobase catalysts may include derivatives of trityl alcohols such as, for example, Malachite green. Photobase catalysts may also include acridine derivatives such as, for example, 9-hydroxy-10-methyl-9-phenyl-9,10-dihydroacridine. Photobase catalysts may also include photoactive carbamate-containing compounds.

The photocatalyst may be present in the compositions and methods described herein in an amount from 0.00050 percent to 30 percent by weight relative to the total weight of the composition.

### Vehicle

The compositions described herein generally include a vehicle suitable for dispersing or dissolving the active material, the photocatalyst, and any other components to facilitate application of the composition onto the substrate surface or into the bulk portions of the substrate. The vehicle may comprise one or more of a solvent, an emulsifier, a surfactant, or other dispersant. The vehicle may also be a physiologically acceptable vehicle A suitable vehicle operates to disperse or dissolve the active material, the photocatalyst, and any other components, and to facilitate application of the composition onto the substrate surface. A suitable vehicle facilitates sufficient contact between the active material and the substrate. In various embodiments, a physiologically acceptable vehicle may be any carrier, solvent, or solvent-containing composition that is suitable for application to physiological tissues such as human hair and human skin, for example. In various embodiments, a physiologically acceptable vehicle is a cosmetically or dermatologically acceptable carrier.

A suitable vehicle may be a solvent. In personal and consumer care product applications, for example, water is a useful solvent. In various embodiments, the compositions described herein may include water in an amount from 1% to 98% by weight relative to the total weight of the composition. Water is also a physiologically acceptable vehicle. Additional solvent or solvent-containing physiologically acceptable vehicles include, but are not limited to, hydroxyl-containing liquids (e.g., alcohols), silicones, oils, hydrocarbons, glycols, ammonium lauryl sulfate, sodium lauryl sulfate, and combinations thereof. In certain embodiments, for example, where the active material is at least partially insoluble in water, other solvents, dispersants, or emulsifiers may be used as physiologically acceptable vehicles, alone or in combination with each other and/or with water.

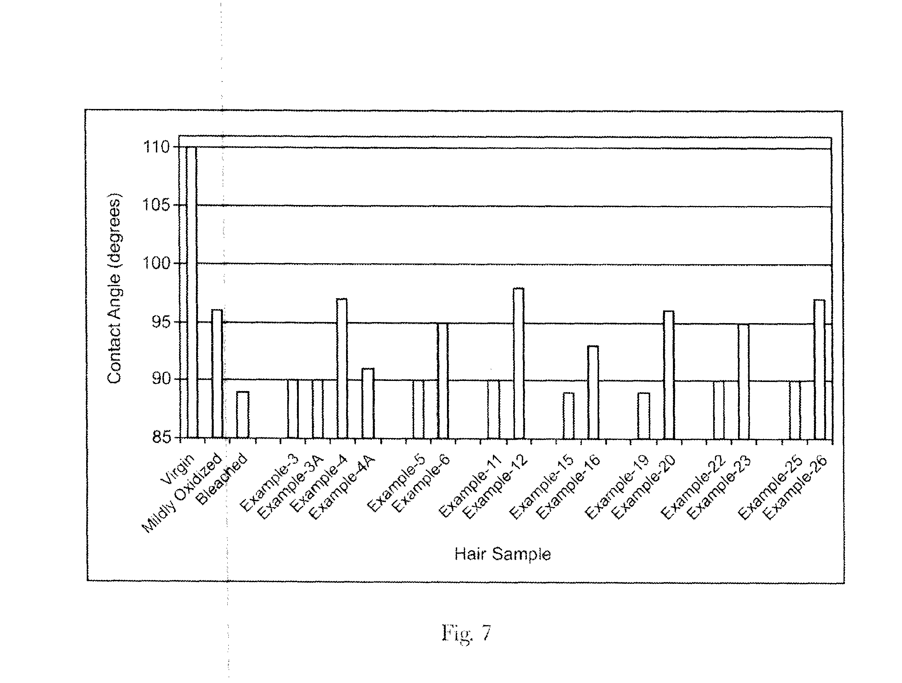

A suitable vehicle is therefore generally used to dilute and/or emulsify the components forming the compositions described herein. A suitable vehicle may dissolve a component (true solution or micellar solution) or a component may be dispersed throughout the vehicle (suspension, dispersion or emulsion). The vehicle of suspension, dispersion or emulsion is typically the continuous phase thereof. That is, other components of the suspension, dispersion or emulsion are distributed on a molecular level or as discrete or agglomerated particles throughout the vehicle. The preparation of such emulsions or dispersions of the active in these cases may be highly important. Small particles contribute to an intimate contact between the active, the substrate and the photoacid catalyst, increasing the reaction rate. For example, in the case of hair surface modification using fatty alcohol and 8-hydroxyquinoline in a water medium, an emulsion that contains very small particles (for example, less than 500 nanometers or more preferably less than 200 nanometers) may be substantially more effective in providing a durable hydrophobic surface than an emulsion containing larger particles (for example, see the data in Figure 7 corresponding to Examples 4 versus 4A).

It will be readily apparent to one of ordinary skill in the art that the appropriate vehicle(s) are dependent upon the specific active material(s), photocatalyst(s), and other optional component(s) used in the compositions described herein.

### Optional Components

The compositions and methods described herein may optionally include a variety of components. For example, in various embodiments, the compositions and methods described herein may include surfactants, emulsifiers, oxidants, reductants, pH regulators, emollients, humectants, proteins, peptides, amino acids, additive polymers, glossers, oils and/or fatty acids, lubricants, sequestrants/chelators, antistatic agents, rheology modifiers, feel agents, fillers, dyes, preservatives, perfumes, other functional components, or combinations thereof. Particular optional components may be found in the CTFA International Cosmetic Ingredient Dictionary, Tenth Edition, 2004; and in McCutcheon, Detergents and Emulsifiers, North American Edition (1986). In various embodiments, the compositions and methods described herein include an oxidizing agent (oxidant). An oxidant may be added, for example, to render a substrate surface more amenable to photocatalytic covalent modification/functionalization. An oxidant may be present in an amount form 0.00050% to 25% by weight relative to the total weight of the composition. Suitable oxidants include, for example, one or more of hydrogen peroxide, urea peroxide, melamine peroxide, percarbonates, alkali metal bromates, perborates, bromates, hypochlorites, chlorites, perchlorates, iodates, periodates, permanganates and persulfates. In certain embodiments, the oxidant is hydrogen peroxide. The identity of the reaction system, the quantities and concentrations of reagents utilized, and the reaction conditions are all dependent, at least in part, upon the substrate to be modified, the active material utilized, and the manner in which the active material is to be associated with the substrate.

### NON-LIMITING EXAMPLES

### EXAMPLE-1: Preparation of Micro-Emulsion of Stearyl Alcohol in Water

A solution of 94.0 grams of deionized water and 2.0 grams of sodium lauryl sulfate is produced in a 200-ml metal beaker. The solution is maintained at a temperature of 80-90°C. 4.0 g of a melt of stearyl alcohol (at 85°C) is added drop-wise to the solution under high shear mixing (using a Silverson® L4RT homogenizer at 6000 rpm) over 15-20 minutes forming a mixture. The mixture is continuously mixed at 80-90°C under high shear for 2 hours. The mixture is then allowed to cool at a rate of 0.5°C per minute to 25°C under high shear mixing. The pH of the mixture is adjusted to approximately 5.5-6.0 using dilute hydrochloric acid solution or dilute sodium hydroxide. The resulting micro-emulsion contains stearyl alcohol with an average particle size of approximately 150 nanometers that is phase-stable over at least six months. Particle size measurements are performed with a Horiba laser scattering particle size distribution analyzer (Model LA-910).

### EXAMPLE-1A: Preparation of Emulsion of Stearyl Alcohol in Water Having Larger Particles

The preparation described in Example-1 is repeated using 1.0 grams of sodium lauryl sulfate instead of 2.0 grams as in Example-1. This modification results is a more opaque emulsion than the micro-emulsion prepared in Example-1. The resulting emulsion contains stearyl alcohol with an average particle size of approximately 1.5 micrometers that is phase-stable over at least two months. Particle size measurements are performed with a Horiba laser scattering particle size distribution analyzer (Model LA-910).

### EXAMPLE-2: Preparation of Prototype Stearyl Alcohol Aqueous Emulsion with Photoacid Catalyst for Hair Treatment

0.0150 g of 8-hydroxyquinoline (8-HQ) is added to the micro-emulsion from Example-1 in a dark room. The resulting emulsion is placed into and stored in an opaque bottle that does not allow any light exposure of the product.

### EXAMPLE-2A: Preparation of Prototype Stearyl Alcohol Aqueous Emulsion with Photoacid Catalyst for Hair Treatment (with Larger Particles)

0.0150 g of 8-HQ is added to the emulsion from Example-1A in a dark room. The resulting emulsion is placed into and stored in an opaque bottle that does not allow any light exposure of the product.

### EXAMPLE-3: Hair Treatment by Spraying Stearyl Alcohol Emulsion

A hair switch* (* = 20 cm long, 4.0-gram) is oxidized with bleach solution, washed and air dried. Under ambient light, the hair switch is thoroughly sprayed with 2.0 g of the emulsion from Example-1. The hair switch is rinsed with tap water for 60 seconds and air dried. The hair switch is then washed with clarifying shampoo (Pantene Pro-V® Clarifying Shampoo), thoroughly rinsed for 3.0 minutes with running tap water, and air dried for at least 5 hours. The washing/rinsing is repeated 6 times. The procedure is repeated with two more identical hair switches (from the same lot).

### EXAMPLE-3A: Hair Treatment by Spraying Larger-Particle Stearyl Alcohol Emulsion

A hair switch* is oxidized with bleach solution, washed and air dried. The hair switch is from the same lot as the hair switch used in Example-3. The hair switch is treated with the emulsion from Example-1A under the same conditions described in Example-3. The procedure is repeated with two more identical hair switches (from the same lot).

### EXAMPLE-4: Hair Treatment by Spraying Prototype Stearyl Alcohol Aqueous Emulsion with Photoacid Catalyst

A hair switch* is oxidized with bleach solution, washed and air dried. The hair switch is from the same lot as the hair switch used in Example-3. The hair switch is treated with the micro-emulsion from Example-2 under the same conditions described in Example-3. The procedure is repeated with two more identical hair switches (from the same lot).

### EXAMPLE-4A: Hair Treatment by Spraying Large-Particle Prototype Stearyl Alcohol Aqueous Emulsion with Photoacid Catalyst

A hair switch* is oxidized with bleach solution, washed and air dried. The hair switch is from the same lot as the hair switch used in Example-3. The hair switch is treated with the emulsion from Example-2A under the same conditions described in Example-3. The procedure is repeated with two more identical hair switches (from the same lot)

### EXAMPLE-5: Hair Treatment by Spreading Stearyl Alcohol Emulsion on Hair

A hair switch* is oxidized with bleach solution, washed and air dried. The hair switch is from the same lot as the hair switches used in Example-3. Under ambient light, 2.0 g of the micro-emulsion from Example-1 is spread onto the hair switch. The spreading process includes addition of small quantities of the micro-emulsion on different spots of the hair surfaces followed by immediate spreading by hand after each addition. The treated hair switch is rinsed with tap water for 60 seconds and air dried. The treated hair switch is then washed with clarifying shampoo (Pantene Pro-V® Clarifying Shampoo), thoroughly rinsed for 3.0 minutes with running tap water, and air dried for at least 5 hours. The washing/rinsing is repeated 6 times. The procedure is repeated with two more identical hair switches (from the same lot).

### EXAMPLE-6: Hair Treatment by Spreading Prototype Stearyl Alcohol Aqueous Emulsion with Photoacid Catalyst on Hair

A hair switch* is oxidized with bleach solution, washed and air dried. The hair switch is from the same lot as the hair switches used in Example-3. The hair switch is treated with the micro-emulsion from Example-2 under exactly the same conditions described in Example-5. The procedure is repeated with two more identical hair switches (from the same lot).

### EXAMPLE-7: Hair Treatment by Dipping in Stearyl Alcohol Emulsion

A hair switch* is oxidized with bleach solution, washed and air dried. The hair switch is from the same lot as the hair switches used in Example-3. In a dark room, the hair switch is dipped into a beaker containing 100.0 g of the emulsion from Example-1. The hair switch is removed from the beaker after 5 minutes and exposed to a bright light (Aquarium 20W Fluorescent tube AquaRays® Model No F20WT12-AR-FS) for 1 minute. The treated hair switch is rinsed with tap water for 60 seconds and air dried. The treated hair switch is then washed with clarifying shampoo (Pantene Pro-V® Clarifying Shampoo), thoroughly rinsed with running tap water for 3.0 minutes, and air dried for at least 5 hours. The washing/rinsing is repeated 6 times. The procedure is repeated with two more identical hair switches (from the same lot).

### EXAMPLE-8: Hair Treatment by Dipping in Prototype Stearyl Alcohol Aqueous Emulsion with Photoacid Catalyst

A hair switch* is oxidized with bleach solution, washed and air dried. The hair switch is from the same lot as the hair switches used in Example-3. The hair switch is treated with the micro-emulsion from Example-2 under exactly the same conditions described in Example-7. The procedure is repeated with two more identical hair switches (from the same lot).

### EXAMPLE-9: Preparation of Micro-Emulsion of Stearyl Alcohol in Water Containing an Oxidizing Agent

A solution of 94.0 grams of deionized water and 2.0 grams of sodium lauryl sulfate is produced in a 200-ml metal beaker. The solution is maintained at a temperature of 80-90°C. 4.0 g of a melt of stearyl alcohol (at 85°C) is added drop-wise to the solution under high shear mixing (using a Silverson® L4RT homogenizer at 6000 rpm) over 15-20 minutes forming a mixture. The mixture is continuously mixed at 80-90°C under high shear for 2 hours. The mixture is then allowed to cool at a rate of 0.5°C per minute to 25°C under high shear mixing. The pH of the mixture is adjusted to approximately 5.5-6.0 using dilute hydrochloric acid solution or dilute sodium hydroxide. 0.030 g of hydrogen peroxide (35% solution) are then added to the mixture. The resulting micro-emulsion contains stearyl alcohol with an average particle size of approximately 150 nanometers that is phase-stable over at least six months. Particle size measurements are performed with a Horiba laser scattering particle size distribution analyzer (Model LA-910).

### EXAMPLE-10: Preparation of Prototype Stearyl Alcohol Aqueous Emulsion with Photoacid Catalyst for Hair Treatment Containing an Oxidizing Agent

0.0150 g of 8-HQ is added to the micro-emulsion from Example-9 in a dark room. The resulting emulsion is placed into and stored in an opaque bottle that does not allow any light exposure of the product.

### EXAMPLE-10A: Preparation of Prototype Stearyl Alcohol Aqueous Emulsion with Photoacid Catalyst for Hair Treatment Containing an Oxidizing Agent and a Preservative System

A prototype stearyl alcohol aqueous emulsion containing hydrogen peroxide, 8-HQ photocatalyst, and a sodium lauryl sulfate vehicle is prepared in a manner analogous to Examples 9 and 10. Additionally, the composition includes a preservative system including three components: 1,2-octanediol; 1,3-dimethylol-5,5-dimethylhydrantoin (DMDMH - Glydant® available from Lonza Inc.); and benzyl alcohol.

### EXAMPLE-11: Hair Treatment by Dipping in Stearyl Alcohol Emulsion

A hair switch* is oxidized with bleach solution, washed and air dried. The hair switch is from the same lot as the hair switches used in Example-3. In a dark room, the hair switch is dipped into a beaker containing 100.0 g of the emulsion from Example-9. The hair switch is removed from the beaker after 5 minutes and exposed to a bright light (Aquarium 20W Fluorescent tube AquaRays® Model No F20WT12-AR-FS) for 1 minute. The treated hair switch is rinsed with tap water for 60 seconds and air dried. The treated hair switch is then washed with clarifying shampoo (Pantene Pro-V® Clarifying Shampoo), thoroughly rinsed with running tap water for 3.0 minutes, and air dried for at least 5 hours. The washing/rinsing is repeated 6 times. The procedure is repeated with two more identical hair switches (from the same lot).

### EXAMPLE-12: Hair Treatment by Dipping in Prototype Stearyl Alcohol Aqueous Emulsion with Photoacid Catalyst Containing an Oxidizing Agent

A hair switch* is oxidized with bleach solution, washed and air dried. The hair switch is from the same lot as the hair switches used in Example-3. The hair switch is treated with the micro-emulsion from Example-10 under exactly the same conditions described in Example-11. The procedure is repeated with two more identical hair switches (from the same lot).

### EXAMPLE-15: Hair Treatment by Dipping in Lauric Acid Aqueous Emulsion

A hair switch* is oxidized with bleach solution, washed, air dried, treated with a reducing agent, washed, and air dried. In a dark room, the hair switch is dipped into a beaker containing 100.0 g of the emulsion from Example-9. The hair switch is removed after 5 minutes and exposed to a bright light (Aquarium 20 W Fluorescent tube AquaRays® Model No F20WT12-AR-FS) for 1 minute, rinsed with tap water for 60 seconds, and air dried. The treated hair switch is then washed with clarifying shampoo (Pantene Pro-V® Clarifying Shampoo), thoroughly rinsed with running tap water for 3.0 minutes, and air dried for at least 5 hours. The washing/rinsing is repeated 6 times. The procedure is repeated with two more identical hair switches (from the same lot).

### EXAMPLE-21: Preparation of Prototype Stearyl Alcohol Aqueous Emulsion Hair Treatment Containing Sulfonated Pyrene and an Oxidizing Agent

In a dark room, 0.0150 g of 8-HQ and 0.03 g of D&C Green 8 (8-hydroxy-1,3,6-pyrenetrisulfonic acid trisodium salt) are added into the micro-emulsion from Example-9 immediately after its preparation. The resulting emulsion is placed into and stored in an opaque bottle that does not allow any light exposure of the product.

### EXAMPLE-24: Preparation of Prototype Stearyl Alcohol Aqueous Emulsion with Photoacid Catalyst Responsive to UV Light

0.10 g of 6-cyano-2-naphthol is added into the micro-emulsion from Example-1 immediately after its preparation. The resulting emulsion is placed into and stored in an opaque bottle that does not allow any light exposure of the product.

### EXAMPLE-27: Hair Contact Angle Determination Method

Two hair fibers randomly extracted from a hair switch are attached on two pairs of anchors (3 cm apart) in such a way that the fibers are 0.20 micrometers apart and parallel to each other under controlled temperature and humidity conditions (approximately 22-25°C and 20-25% humidity). A 0.20 microliter deionized water droplet (±0.02 microliter) is placed onto the two hair fibers with a micro syringe. A video image of the water droplet is taken from the side using a digital camera/software (Model FTA32). The initial contact angle of the droplet on the hair fiber is measured. A typical determination of a treatment includes averaging of 60 contact angle measurements for three switches, four fiber pairs for each switch, and five places across each fiber pair. The error of the method is approximately 1-2%. The contact angles of treated hair from Examples 3, 4, 5, 6, 11, 12, 15, B, and C are measured using this method. Benchmark contact angle data of hair of various natures (virgin, mildly oxidized, and bleached) are also measured for comparison. The corresponding results of the measurements are given in Table 1 and FIG. 7.

**TABLE 1: Hair Contact**

| Angles | |
|---|---|
| Hair Sample | Contact Angle (degrees) |
| Virgin | 110 |
| Mildly Oxidized | 96 |
| Bleached | 89 |
| Example-3 | 90 |
| Example-3A | 90 |
| Example-4 | 97 |
| Example-4A | 91 |
| Example-5 | 90 |
| Example-6 | 95 |
| Example-11 | 90 |
| Example-12 | 98 |
| Example-15 | 89 |
| Example 81 | 93 |
| Example 82 | 90 |

### EXAMPLE-28: Rinse-off Conditioning Treatment of Hair Switches from Example-3

All three hair switches from Example-3 are treated with silicone-containing, rinse-off hair conditioner (Pantene Pro-V® Always Smooth; 0.1 mL of conditioner for 1.0 g of hair) and then rinsed with running tap water for 30 seconds and air dried for at least 5 hours.

### EXAMPLE-29: Rinse-off Conditioning Treatment of Hair Switches from Example-4

All three hair switches from Example-4 are treated with silicone-containing, rinse-off hair conditioner (Pantene Pro-V® Always Smooth; 0.1 mL of conditioner for 1.0 g of hair) and then rinsed with running tap water for 30 seconds and air dried for at least 5 hours. An evaluation of the hair switches from Example-29 relative to the hair switches from Example-28 is provided in Table 2.

**Table 2: Rinse-off Conditioning Evaluation**

| PROPERTY | HAIR FROM EXAMPLE-29 | HAIR FROM EXAMPLE-28 |
|---|---|---|
| Quantity of Silicone Deposition from Conditioner on Hair | Higher | Lower |
| Dry Hair Friction | Lower | Higher |
| Combability | Higher | Lower |
| Mechanical damage | Lower | Higher |
| Shine | Higher | Lower |

The hair from Example-29 has lower dry friction and mechanical damage than the hair from Example 28. The hair from Example-29 has higher levels of deposited silicones on the hair surface than the hair from Example-28. Combability and shine are also enhanced in the hair from Example-29 relative to the hair from Example-28.

### EXAMPLE-30: Preparation of a Glycerin Solution

A glycerin solution is prepared by adding 90.0 grams of deionized water 10 grams of glycerin into a 200-ml glass beaker and mixed with a magnetic stirrer for 5 minutes.

### EXAMPLE-31: Preparation of a Prototype Hair Moisturizer Containing Photoacid Catalyst

0.0150 g of 8-HQ and 0.030 g of hydrogen peroxide (35% solution) are added into the solution from Example-30 in a dark room. The resulting solution is placed into and stored in an opaque bottle that does not allow any light exposure of the product.

### EXAMPLE-32: Hair Treatment by Spraying Glycerin Solution

A hair switch* is oxidized with bleach solution, washed and air dried. Under ambient light, the hair switch is thoroughly sprayed with 2.0 g of the solution from Example-30. The treated hair switch is rinsed with tap water for 60 seconds and air dried. The treated hair switch is then washed with clarifying shampoo (Pantene Pro-V® Clarifying Shampoo), thoroughly rinsed with running tap water for 3.0 minutes, and air dried for at least 5 hours. The washing/rinsing is repeated 2 times. The procedure is repeated with two more identical hair switches (from the same lot).

### EXAMPLE-33: Hair Treatment by Spraying the Prototype Humectant

A hair switch* is oxidized with bleach solution, washed and air dried. The hair switch is from the same lot as the hair switches used in Example-32. The hair switch is treated with the prototype moisturizer from Example-31 under exactly the same conditions described in Example-32. The procedure is repeated with two more identical hair switches (from the same lot). Evaluation of the hair switches from Example-33 shows lower rate of moisture loss compared to hair switches from Example-32 when humidity is decreased from 70% to 20%. When formulations from Examples-32 and 33 are evaluated by consumer panel (via spray devise), it is concluded that formulation from Example-33 contributes to softer hair.

### EXAMPLE-34: Preparation of a Dye Solution

A dye solution is prepared by adding 100 g of deionized water and 0.50 gram of Direct Dye 243 (Red BWS supplied by Aakash Chemicals & Dye-Stuffs, Inc.) into a 200-ml glass beaker. The solution is mixed with a magnetic stirrer for 10 minutes to a dark red solution. Direct Dye 243:

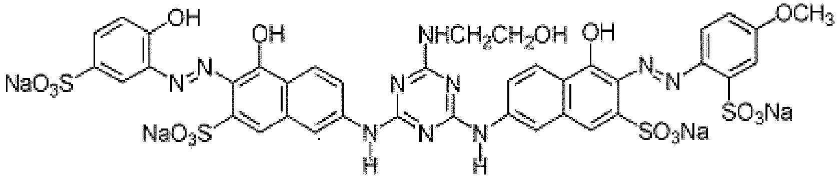

### EXAMPLE-35: Preparation of a Prototype Hair Dye Solution with Photoacid Catalyst

0.0150 g of 8-HQ are added into the solution from Example-34 in a dark room. The resulting solution is placed into and stored in an opaque bottle that does not allow any light exposure of the product.

### EXAMPLE-36: Hair Treatment by Dipping in a Dye Solution

A hair switch* is oxidized with bleach solution, washed and air dried. In a dark room, the hair switch is dipped into a 200-ml glass beaker containing 100 g of the dye solution from Example-34 for 10 minutes. The treated hair switch is then removed from the dye solution, rinsed under running tap water for 60 seconds, and air dried. The dried hair switch is then washed with clarifying shampoo (Pantene Pro-V® Clarifying Shampoo), rinsed with running tap water for 3.0 minutes, and air dried for at least 5 hours. The shampoo washing/rinsing cycle is repeated 3 more times. The color of the hair switch is then measured via Color Computer (Microflash® by Datacolor) and compared to the color of the initial (untreated) hair switch. This difference in color between the treated and untreated switches is expressed as DeltaE. The procedure is repeated with two more identical hair switches (from the same lot).

### EXAMPLE-37: Hair Treatment by Dipping in a Prototype Hair Dye Solution Containing Photoacid Catalyst

A hair switch* is oxidized with bleach solution, washed and air dried. The hair switch is from the same lot as the hair switches used in Example-36. The hair switch is dipped into the prototype dye solution from Example-35 and treated under exactly the same conditions described in Example-36. The color of the hair switch is then measured via Color Computer (Microflash® by Datacolor) and compared to the color of the initial (untreated) hair switch. This difference in color between the treated and untreated switches is expressed as DeltaE. The procedure is repeated with two more identical hair switches (from the same lot). Table provides the average results (for three switches) of the color evaluation for treatments of Examples 36 and 37. Treatment of Example-37 results in more effective hair dying (higher DeltaE from the original hair).

**Table: Color Difference Between Treated and Initial Bleached Hair**

| SAMPLE | Delta E |
|---|---|
| EXAMPLE-36 | 4 |
| EXAMPLE-37 | 6 |

### EXAMPLE 38: Treatment Of Dyed Hair Switches With Prototype Stearyl Alcohol Emulsion Containing Photocatalyst And Determination Of Fading After Shampoo Washes

Five virgin hair switches* A, B, and C (from the same batch) are dyed with a permanent oxidative dye (Koleston Perfect Shade Red 77/44; level 3) according to the product instructions. After air drying, the switches are treated with 2.0 grams of the prototype emulsion from Example 2 by spraying, rinsed with water, washed with clarifying shampoo and then thoroughly rinsed again with water and air-dried (as described in Example 3). Switch A is measured for color using color computer Minolta and retained for future comparison. Switch B is washed with Wella Lifetex color protection shampoo (using 0.10 ml of shampoo per gram of hair) and rinsed with water of 37°C and flow rate of 1.0 gallon per minute for 30 seconds. After rinsing, the switch is squeezed once between fingers to remove excess water and blow-dried on high heat for 3.0 minutes and then measured with a color computer. The shampoo-washing/color measurement procedure is repeated five more times (one washing every day). The same shampoo washing protocol (followed by color measurement) is applied for switch C for 12 days (one each day).

### EXAMPLE 41: Preparation Of A Control Perfume Emulsion

A solution of 94.0 grams of deionized water and 2.0 grams of sodium lauryl sulfate is produced in a 200-ml metal beaker. The solution is maintained at a temperature of 80-90°C. Five grams of 2-hydroxyethyl benzene is added over 15 minutes to the solution under high shear mixing (using a Silverson® L4RT homogenizer at 6000 rpm). The mixture is continuously mixed at 80-90°C under high shear for 2 hours. The mixture is then allowed to cool at a rate of 0.5°C per minute to 25°C under high shear mixing. The pH of the mixture is adjusted to approximately 5.5-6.0 using dilute hydrochloric acid solution or dilute sodium hydroxide.

### EXAMPLE 45: Preparation of Aqueous Solution of Cyclodexrin

Into solution of 199.1 grams of deionized water and 0.30 grams of anionic surfactant Dowfax® 3B2 are added under stirring 0.30 grams of beta-cyclodextrin and 0.30 grams hydroxypropyl beta-cyclodextrin having an average degree of substitution of 3.3. The pH of the mixture is adjusted to approximately 5.5-6.0 using dilute hydrochloric acid solution or dilute sodium hydroxide.

### EXAMPLE-49: Preparation of a Polyvinyl Alcohol Solution

A polyvinyl alcohol solution is prepared by adding 4.0 grams of polyvinyl alcohol (Gohsenol® GH-23, available from Nippon Gohsei) to 96.0 grams of deionized water over 10 minutes under stirring at 85°C until a clear solution develops. The solution is left to cool to room temperature before use.

### EXAMPLE-50: Preparation of a Prototype Hair Styling Primer Containing Photoacid Catalyst

0.0150 g of 8-HQ and 0.030 g of hydrogen peroxide (35% solution) are added into the solution from Example-38 in a dark room. The resulting solution is placed into and stored in an opaque bottle that does not allow any light exposure of the product.

### EXAMPLE-50A: Preparation of a Prototype Hair Styling Primer Containing Photoacid Catalyst 0.0150 g of 8-HQ are added into the solution from Example-38 in a dark room. The resulting solution is placed into and stored in an opaque bottle that does not allow any light exposure of the product.

### EXAMPLE-51: Hair Treatment by Spraying Polyvinyl Alcohol Solution

A hair switch* is oxidized with bleach solution, washed and air dried. Under ambient light, the hair switch is thoroughly sprayed with 2.0 g of the solution from Example-38. The hair switch is rinsed with tap water for 60 seconds and air dried. The hair switch is then washed with clarifying shampoo (Pantene Pro-V® Clarifying Shampoo), thoroughly rinsed with running tap water for 3.0 minutes, and air dried for at least 5 hours. The washing/rinsing is repeated. The procedure is repeated with two more identical hair switches (from the same lot).

### EXAMPLE-52: Hair Treatment by Spraying the Prototype Hair Styling Primer

A hair switch* is oxidized with bleach solution, washed and air dried. The hair switch is from the same lot as the hair switches used in Example-51. The hair switch is treated with the prototype hair styling primer from Example-50 under exactly the same conditions described in Example 51. The procedure is repeated with two more identical hair switches (from the same lot).

### EXAMPLE-52A: Hair Treatment by Spraying the Prototype Hair Styling Primer

A hair switch* is oxidized with bleach solution, washed and air dried. The hair switch is from the same lot as the hair switches used in Example-51. The hair switch is treated with the prototype hair styling primer from Example-52A under exactly the same conditions described in Example 51. The procedure is repeated with two more identical hair switches (from the same lot).

### EXAMPLE-52B: Comparison of Hair Switches from Example-51 with Hair Switches from Example-52 and Example-52A

Visual and sensory inspection of the switches from Example-51, Example-52, and Example-52A shows that hair switches from Example-52 and Example-52A are smoother and shinier than hair switches from Example-51, especially near the hair tips.

### EXAMPLE-53: Treatment of Hair Switches from Example-51 with a Styling Mousse

All three hair switches from Example-51 are treated while wet with a styling mousse. The hair switches are combed twice using a large tooth comb and combed once using a small tooth comb. The hair switches are lightly blotted with paper towel and the mousse is added using a syringe (0.25 grams of mousse per gram of hair). The mousse is spread throughout the hair and the hair is massaged with the mousse. The mousse has the following composition in weight percent: 72.73% water; 2.0% Luviskol® VA 73W (50% PVP/VA copolymer available from BASF); 10.0% Carbowax® 600 (PEG-12 available from Dow); 2.0% cyclopentasiloxane (SF1202 available from Momentive); 2.0% silicone emulsion (50% dimethicone emulsion available from Toray Silicones); 0.45% isosteareth-20; 0.50% benzyl alcohol; 0.2% methyl paraben; 0.12% disodium EDTA; and 10.0% Propellant Aeron® A-70 (propane/isobutene available from Diversified CPC). The mousse is prepared by mixing all the ingredients, except the propellant, into a uniform mixture using a Silverson® L4RT homogenizer (5000 rpm) at 48-52°C. The mixture is then cooled to 25°C, filled into an aerosol container, and the aerosol propellant is added.

### EXAMPLE-54: Treatment of Hair Switches from Example-52with a Styling Mousse

All three hair switches from Example 52 and Example-52A are treated in exactly the same protocol as described in Example-53.

### EXAMPLE-55: Shape Retention Evaluation of Hair Switches from Examples 53 and 54

Hair switches from Examples 53 and 54 are fixated around 0.5 inch Teflon® cylindrical rods and dried. The dried hair switches are removed from the rod. The hair curl possesses an initial length (L₀). A small weight is applied to the hair switches and their lengths (L₁) are measured. The hair switches are then allowed to relax to a final length (L₂). The percent recoveries of the hair switches are calculated as (L₁-L₂)/(L₁-L₀). Hair switches from Example-54 have a higher percent recovery than hair switches from Example-53.

### EXAMPLE-56: Hair Treated with Ethylene Carbonate and Photoacid Catalyst Solution to Increase Rigidity

The initial rigidity of six individual hair fibers is measured. The fibers are then soaked in a solution of 20% (w/v) ethylene carbonate and 2% (w/v) 6-cyano-2-naphthol in 3:2 methanol hexane for 30 minutes without light exposure at wavelengths < 550nm. After soaking, the hair fibers are exposed to a light source with significant light intensity at the wavelengths absorbed by 6-cyano-2-naphthol (approximately 300nm - 350nm). After exposure, the hair fibers are rinsed, allowed to dry and the rigidity of the hair measured again. The average rigidity of the six hair fibers shows an increase of 44%. Scanning electron microscopy (SEM) of the hair fibers shows that the surface of the fibers is unchanged.

### EXAMPLE-57: Hair Treated with Poly(acrylic acid) and Photoacid Catalyst Solution to Increase Rigidity

The initial rigidity of six individual hair fibers is measured. The fibers are then soaked in a solution of 20% (w/v) poly(acrylic acid) (molecular weight approximately 10,000) and 2% (w/v) 6-cyano-2-naphthol in 3:2 methanol hexane for 30 minutes without light exposure at wavelengths < 550nm. After soaking, the hair fibers are exposed to a light source with significant light intensity at the wavelengths absorbed by 6-cyano-2-naphthol (approximately 300nm - 350nm). After exposure, the hair fibers are rinsed, allowed to dry, and the rigidity of the hair measured again. The average rigidity of the six hair fibers shows an increased of 56%. SEM of the hair fibers shows that the surface of the fibers is unchanged.

### EXAMPLE-58: Hair Treated with Ethyl Oxazoline and Photoacid Catalyst Solution to Increase Rigidity

The initial rigidity of six individual hair fibers is measured. The fibers are then soaked in a solution of 20% (w/v) ethyl oxazoline and 2% (w/v) 6-cyano-2-naphthol in 3:2 methanol hexane for 30 minutes without light exposure at wavelengths < 550nm. After soaking, the hair fibers are exposed to a light source with significant light intensity at the wavelengths absorbed by 6-cyano-2-naphthol (approximately 300nm - 350nm). After exposure, the hair fibers are rinsed, allowed to dry, and the rigidity of the hair measured again. The average rigidity of the six hair fibers shows an increase of 30%. SEM of the hair fibers shows that the surface of the fibers is unchanged.

### EXAMPLE-59: Hair Treated with a Reducing Agent

An approximately 4g virgin hair switch is treated with an ammonium thioglycolate 5% solution (35-°C; pH 9.6) for 10 minutes and thoroughly rinsed with warm water. The hair switch is then soaked for 10 minutes in a 1% aqueous hydrochloric acid solution and thoroughly rinsed with warm water for 10 minutes and air-dried.

### EXAMPLE-60: Preparation of α-Methyl Styrene and Photoacid Catalyst Solution

A solution comprising α-Methyl Styrene and a photoacid catalyst is produced by adding 90.0 grams of methanol/acetone (1:1), 2.0 grams of 6-cyano-2-naphthol, and 8.0 grams of alpha-methyl styrene into a 200 ml beaker. The ingredients are mixed for 5 minutes to produce a clear solution in a dark room. The solution is then stored in an opaque bottle that does not allow any light exposure of the product.

### EXAMPLE-61: Reduced Hair Treated with α-Methyl Styrene and Photoacid Catalyst Solution to Increase Rigidity

The hair switch from Example-59 is soaked in 100 ml of the solution from Example-60 in a dark room for 10 minutes. The hair switch is then removed from the beaker and exposed to a light source with substantial light intensity at the wavelengths absorbed by 6-cyano-2-naphthol (approximately 300-350 nm). After exposure, the hair fibers are rinsed and allowed to air dry. The stiffness (determined as resistance to bending) of hair fibers treated with the protocol of Example-61 is 50% higher than untreated hair fibers.

### EXAMPLE-65: Poly(acrylic acid) and Photoacid Catalyst

A solution of 1% (w/v) poly(acrylic acid) and 100 ppm 8-HQ.

### EXAMPLE-66: Poly(vinyl pyrrolidone-co-acrylic acid) and Photoacid Catalyst

A solution of 1% (w/v) poly(vinyl pyrrolidone-co-acrylic acid) and 100 ppm 8-HQ.

### EXAMPLE-67: Butane Tetracarboxylic Acid and Photoacid Catalyst

A solution of 1% (w/v) butane tetracarboxylic acid and 100 ppm 8-HQ.

### EXAMPLE-68: Citric Acid and Photoacid Catalyse

A solution of 1% (w/v) citric acid and 100 ppm 8-HQ.

### EXAMPLE-69: Poly(acrylic acid) and Photoacid Catalyst

A solution of 1% (w/v) poly(acrylic acid) and 100 ppm 8-HQ.

### EXAMPLE-76: Poly(styrene sulfonate-co-acrylic acid) and Photoacid Catalyst

0.1% Poly(styrene sulfonate-co-acrylic acid) and 100ppm of 8-HQ are dissolved in water.

### EXAMPLE 78: To the base formulation is added 100ppm of 8-HQ and 1% poly (acrylic acid-co-styrene sulphonate).

### EXAMPLE 79: To the base formulation is added 100ppm of 8-HQ and 1% poly (vinyl alcohol).

### EXAMPLE 80: To the base formulation is added 100ppm of 8-HQ and 1% stearic acid.

### EXAMPLE 81: Preparation of a rinse-off hair conditioner containing 8-HQ

Into 100 g of conditioner with the composition given below, a quantity of 0.0150 g of 8-HQ is added and mixed for 10 minutes at room temperature. The mixture is then placed into an opaque bottle that does not allow any light exposure of the product.

### Conditioner Composition A1

Stearamidopropyldimethylamine (2.00%); L-Glutamic acid (0.64%); Cetyl alcohol (6.00%); Stearyl alcohol (4.00%); Dimethicone/cyclomethicone mixture (3.00%); Kathon CG (0.03%); Benzyl alcohol (0.50%); Methyl paraben (0.20%); Propyl paraben (0.10%); Disodium EDTA (0.13%); Perfume (0.50%); Water (82.90%).

### EXAMPLE 82: Treatment of bleached hair with rinse-off conditioner from Example A

A hair switch* is oxidized with bleach solution, washed and rinsed. A quantity of 0.4 ml of conditioner from Example A (containing 8-HQ) is added, massaged into hair for 30 seconds under ambient light, and rinsed with water. The hair switch is then washed with clarifying shampoo (Pantene Pro-V® Clarifying Shampoo), thoroughly rinsed for 3.0 minutes with running tap water, and air dried for at least 5 hours. The washing/rinsing is repeated 5 more times. The procedure is repeated with two more identical hair switches (from the same lot).

### EXAMPLE 83: Treatment of bleached hair with rinse-off conditioner composition A1

A hair switch* is oxidized with bleach solution, washed and rinsed. A quantity of 0.4 ml of conditioner composition A1 (not containing 8-HQ) is added, massaged into hair for 30 seconds under ambient light, and rinsed with water. The hair switch is then washed with clarifying shampoo (Pantene Pro-V® Clarifying Shampoo), thoroughly rinsed for 3.0 minutes with running tap water, and air dried for at least 5 hours. The washing/rinsing is repeated 5 more times. The procedure is repeated with two more identical hair switches (from the same lot as Example 82).

### CLAUSES: The following clauses are part of the description:

### Clause:

1. A personal care composition characterized in that it comprises:
   (a) an active material having one or more functional groups capable of covalent attachment in the presence of an acid or a base to one or more complementary functional groups;
   (b) a photocatalyst capable of generating an acid or a base upon exposure to light; and
   (c) a physiological acceptable vehicle for dispersing or dissolving the active material and the photocatalyst for application of the composition to a substrate; and
   wherein the vehicle is a physiological acceptable vehicle and the substrate is selected from the group consisting ofhair, skin, nail, teeth and combinations thereof.
2. The composition recited in clause 1 further comprising a component selected from the group consisting of a surfactant, an emulsifier, an oxidant, a pH controlling component, a feel agent, a rheology modifier, a filler, a perfume, and combinations thereof.
3. The composition recited in clause 1 wherein the vehicle comprises a solvent, and wherein the solvent is selected from the group consisting of water, silicones, oils, hydrocarbons, lauryl sulfate salts and combinations thereof.
4. The composition recited in clause 1 wherein the photocatalyst is a photoacid selected from the group consisting of aromatic hydroxyl compounds, sulfonated pyrene compounds, onium salts, diazomethane derivatives, bissulfone derivatives, disulfuno derivatives, nitrobenzyl sulfonate derivatives, sulfonic acid ester derivatives, sulfonic acid esters of N-hydroxyimides, and combinations thereof, wherein the light absorbed by the photocatalyst is selected from the group consisting of ultraviolet light, visible light, and combinations thereof, and wherein the photocatalyst is present in an amount of from 0.00050% to 10% by weight relative to the total weight of the composition.
5. The composition recited in clause 4 wherein the aromatic hydroxy compound is a hydroxyl-substituted quinoline, preferably wherein the hydroxyl-substituted quinoline is 8-hydroxyquinoline.
6. The composition recited in clause 1 wherein the photocatalyst is a photobase selected from the group consisting of hydroxyl-substituted quinolines, trityl alcohol derivatives and acridine derivatives, preferably wherein the photobase is a hydroxyl-substituted quinoline
7. The composition recited in clause 6 wherein the photobase is 8-hydroxyquinoline.
8. The composition recited in clause 1 wherein the active material is a hydrophobic material and the composition further comprises one or both of a surfactant and an emulsifier, preferably wherein the active material is selected from the group consisting of a fatty acid, a fatty alcohol, a fatty amine, an aminosilicone, a polyvinyl alcohol, a polyvinyl alcohol-polyvinyl pyrrolidone copolymer, a polycaprolactone, an optical brightener, a humectant, a silanol, a dimethylsilicone functionalized with one or more of primary, secondary, carboxyl or hydroxyl functional groups, a malodor absorber/remover, a perfume, and combinations thereof.
9. The composition recited in clause 1 wherein the active material is selected from the group consisting of glycerin, hair coloring, a dye, stearyl alcohol, lauric acid, direct dye 243, ethylene carbonate, poly(acrylic acid), ethyl oxazoline, poly(styrene), poly(vinyl pyrrolidone-co-acrylic acid), butane tetracarboxylic acid, citric acid, poly(styrene sulfonate-co-acrylic acid), an ethyl ester of PVM/MA copolymer, ethoxylated poly(dimethylsiloxane), cyclodextrin, cyclodextrin derivative, and combinations thereof.
10. The composition recited in clause 1 wherein the personal care composition is selected from the group consisting of lipstick, mascara, rouge, foundation, blush, eyeliner, lipliner, lip gloss, body powder, sunscreen, sun block, nail polish, mousse, spray, styling gel, nail conditioner, bath gel, shower gel, shampoo, cream rinse, hair dye, hair coloring product, hair conditioner, hair shine product, hair anti-frizz product, malodor absorber/remover, lip balm, skin conditioner, cold cream, moisturizer, hair spray, soap, body scrub, exfoliant, astringent, depilatory, permanent waving solution, antidandruff formulation, antiperspirant composition, deodorant, shaving product, preshaving product, after shaving product, cleanser, skin gel, and rinse.
11. The composition recited in clause 1, wherein the active material comprises stearyl alcohol, the photocatalyst comprises 8-hydroxyquinoline, the vehicle comprises sodium lauryl sulfate, and the composition further comprises water and hydrogen peroxide.
12. A method for treating a substrate comprising:
   applying at least one active material to the substrate, the active material having one or more functional groups, and the substrate having one or more complementary functional groups;
   applying to the substrate at least one photocatalyst capable of generating an acid or base on exposure to light; and
   exposing the photocatalyst and the at least one active material to light for forming covalent attachments between the one or more functional groups of the at least one active material and a reagent selected from the group consisting of a second active material, a substrate and a combination thereof.
13. The method of Clause 12, wherein the covalent attachment is between the one or more functional groups of the at least one active material and a second active material to form a product.
14. The method of Clause 13, wherein the product is further reacted with a substrate.
15. The method of Clause 12, wherein the covalent attachments is an esterification reaction between the one or more functional groups of the at least one active material and the substrate.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm."

## Claims

1. A personal care composition **characterized in that** it comprises:
(a) an active material having one or more functional groups capable of covalent attachment to a substrate in the presence of an acid or a base to one or more complementary functional groups;
(b) a photocatalyst capable of generating an acid or a base upon exposure to light; and
(c) a physiological acceptable vehicle for dispersing or dissolving the active material and the photocatalyst for application of the composition to a substrate; and
wherein the vehicle is a physiological acceptable vehicle and the substrate is selected from the group consisting ofhair;
wherein the active material is selected from the group consisting of glycerin, ethylene carbonate, poly(acrylic acid), ethyl oxazoline, poly(styrene), poly(vinyl pyrrolidone-co-acrylic acid), butane tetracarboxylic acid, citric acid, poly(styrene sulfonate-co-acrylic acid), an ethyl ester of PVM/MA copolymer, ethoxylated poly(dimethylsiloxane), cyclodextrin, cyclodextrin derivative, and combinations thereof;
wherein the photocatalyst is an aromatic hydroxyl compound.

2. The composition recited in claim 1 further comprising a component selected from the group consisting of a surfactant, an emulsifier, an oxidant, a pH controlling component, a feel agent, a rheology modifier, a filler, a perfume, and combinations thereof.

3. The composition recited in claim 1 wherein the vehicle comprises a solvent, and wherein the solvent is selected from the group consisting of water, silicones, oils, hydrocarbons, lauryl sulfate salts and combinations thereof.

4. The composition recited in claim 1 wherein the light absorbed by the photocatalyst is selected from the group consisting of ultraviolet light, visible light, and combinations thereof, and wherein the photocatalyst is present in an amount of from 0.00050% to 10% by weight relative to the total weight of the composition.

5. The composition recited in any of the preceding claims wherein the aromatic hydroxy compound is a hydroxyl-substituted quinoline, preferably wherein the hydroxyl-substituted quinoline is 8-hydroxyquinoline.

6. The composition recited in claim 1 wherein the active material is a hydrophobic material and the composition further comprises one or both of a surfactant and an emulsifier, preferably wherein the active material is selected from the group consisting of a fatty acid, a fatty alcohol, a fatty amine, an aminosilicone, a polyvinyl alcohol, a polyvinyl alcohol-polyvinyl pyrrolidone copolymer, a polycaprolactone, an optical brightener, a humectant, a silanol, a dimethylsilicone functionalized with one or more of primary, secondary, carboxyl or hydroxyl functional groups, a malodor absorber/remover, a perfume, and combinations thereof.

7. The composition recited in claim 1 wherein the personal care composition is selected from the group consisting of mascara, mousse, spray, styling gel, shampoo, cream rinse, hair conditioner, hair shine product, hair anti-frizz product, hair spray, permanent waving solution, antidandruff formulation,.

8. The composition recited in claim 1, wherein the active material comprises stearyl alcohol, the photocatalyst comprises 8-hydroxyquinoline, the vehicle comprises sodium lauryl sulfate, and the composition further comprises water and hydrogen peroxide.

9. A method for treating a substrate comprising:
applying at least one active material to the substrate, the active material having one or more functional groups, and the substrate having one or more complementary functional groups;
applying to the substrate at least one photocatalyst capable of generating an acid or base on exposure to light; and
exposing the photocatalyst and the at least one active material to light for forming covalent attachments between the one or more functional groups of the at least one active material and a reagent selected from the group consisting of a second active material, a substrate and a combination thereof;
wherein the active material is selected from the group consisting of glycerin, ethylene carbonate, poly(acrylic acid), ethyl oxazoline, poly(styrene), poly(vinyl pyrrolidone-co-acrylic acid), butane tetracarboxylic acid, citric acid, poly(styrene sulfonate-co-acrylic acid), an ethyl ester of PVM/MA copolymer, ethoxylated poly(dimethylsiloxane), cyclodextrin, cyclodextrin derivative, and combinations thereof;
wherein the photocatalyst is an aromatic hydroxyl compound.

10. The method of Claim 9, wherein the covalent attachment is between the one or more functional groups of the at least one active material and a second active material to form a product.

11. The method of Claim 10, wherein the product is further reacted with a substrate.

12. The method of Claim 9, wherein the covalent attachments is an esterification reaction between the one or more functional groups of the at least one active material and the substrate.

13. Use of the composition according to any of claims 1 to 8 for treating hair.

14. A mechanism of use comprising:
(i) a reagent solution is provided that includes a reagent being an active component, and a photo acid catalyst;
(j) the reagent solution is applied to a substrate being hair
(k) the components of the reagent solution deposit on the surface of the substrate;
(l) the system comprising the reagent solution and the substrate is exposed to light and the light causes the deprotonation of the photocatalyst;
(m) a photoacid-catalyzed esterification reaction occurs between the reagent and the substrate surface;
(n) un-reacted catalyst, reagent, and protons diffuse from the substrate surface and are removed from the system;
(o) the modified/functionalized substrate is dried;
the modified/functionalized substrate is washed and rinsed and the modified/functionalized substrate substantially retains the covalently bound reagent after washing and rinsing.
